Europäisches Patentamt

European Patent Office

Office européen des brevets

(19)

(11) **EP 1 294 941 B1**

(12) **FASCICULE DE BREVET EUROPEEN**

(45) Date de publication et mention
de la délivrance du brevet:
**05.01.2005 Bulletin 2005/01**

(21) Numéro de dépôt: **01938296.9**

(22) Date de dépôt: **22.05.2001**

(51) Int Cl.⁷: **C12Q 1/68**, G01N 33/574

(86) Numéro de dépôt international:
**PCT/FR2001/001566**

(87) Numéro de publication internationale:
**WO 2002/000929 (03.01.2002 Gazette 2002/01)**

(54) **PROCEDE DE DIAGNOSTIC IN VITRO DU CANCER DE LA PROSTATE ET KIT DE MISE EN OEUVRE**

DIAGNOSTISCHES IN VITRO-VERFAHREN FÜR PROSTATAKREBS SOWIE HIERFÜR GEEIGNETES TESTSYSTEM

METHOD FOR IN VITRO DIAGNOSIS OF PROSTATIC CANCER AND KIT THEREFOR

(84) Etats contractants désignés:
**AT BE CH CY DE DK ES FI FR GB GR IE IT LI LU MC NL PT SE TR**

(30) Priorité: **27.06.2000 FR 0008266**

(43) Date de publication de la demande:
**26.03.2003 Bulletin 2003/13**

(73) Titulaire: **Urogene**
**91000 Evry (FR)**

(72) Inventeurs:
• **CUSSENOT, Olivier**
  **F-75020 Paris (FR)**
• **LATIL, Alain**
  **F-78350 Jouy en Josas (FR)**
• **CHANTREL-GROUSSARD, Karine**
  **F-78220 Viroflay (FR)**

(74) Mandataire: **Vuillermoz, Bruno**
**Cabinet Laurent & Charras**
**B.P. 32**
**20, rue Louis Chirpaz**
**69131 Ecully Cédex (FR)**

(56) Documents cités:
WO-A-97/15686        WO-A-98/46795
WO-A-99/56779        US-A- 6 140 049

• LATIL A ET AL.: "Loss of heterozygosity at chromosome 16q in prostate adenocarcinoma: Identification of three independent regions" CANCER RESEARCH, vol. 57, 1997, pages 1058-1062, XP002168426 cité dans la demande

• LATIL A ET AL.: "Loss of heterozygosity at 7q31 is a frequent and early event in prostate cancer" CLINICAL CANCER RESEARCH, vol. 1, 1995, pages 1385-1389, XP000993258 cité dans la demande

• LATIL A ET AL.: "Loss of heterozygosity at chromosome arm 13q and RB1 status in human prostate cancer" HUMAN PATHOLOGY, vol. 30, no. 7, 1999, pages 809-815, XP001001282 cité dans la demande

• LATIL A ET AL.: "Genetic alterations in localized prostate cancer: Identification of a common region of deletion on chromosome arm 18q" GENES, CHROMOSOMES & CANCER, vol. 11, 1994, pages 119-125, XP001001285

• PESCHE S ET AL.: "PTEN/MMAC1/TEP1 involvement in primary prostate cancers." ONCOGENE, vol. 16, juin 1998 (1998-06), pages 2879-2883, XP001025516

• UEDA T ET AL.: "Identification of a 1-cM region of common deletion on 13q14 associated with human prostate cancer" GENES, CHROMOSOMES & CANCER, vol. 24, 1999, pages 183-190, XP001001291 cité dans la demande

• LATIL A ET AL.: "VEGF overexpression in clinically localized prostate tumors and neuropilin-1 overexpression in metastatic forms" INTERNATIONAL JOURNAL OF CANCER, vol. 89, mars 2000 (2000-03), pages 167-171, XP001025512

- LATIL A ET AL.: "Differential chromosome allelic imbalance in the progression of human prostate cancer" THE JOURNAL OF UROLOGY, vol. 156, 1996, pages 2079-2083, XP001001284 cité dans la demande
- JENKINS R ET AL.: "Prognostic significance of allelic imbalance of chromosome arms 7q, 8p, 16q, and 18q in stage T3N0Mo prostate cancer" GENES, CHROMOSOMES & CANCER, vol. 21, 1998, pages 131-143, XP001025523
- BI CHE I ET AL.: "Quantitation of hTERT gene expression in sporadic breast tumors with a real-time reverse transcription-polymerase chain reaction" CLINICAL CANCER RESEARCH, vol. 6, 2000, pages 452-459, XP000993257
- DATABASE WPI Section Ch, Week 199901 Derwent Publications Ltd., London, GB; Class B04, AN 1999-002833 XP002168428 & JP 10 276780 A (SUMITOMO METAL IND LTD), 20 octobre 1998 (1998-10-20)
- CHENG L ET AL.: "Allelic imbalance in the clonal evolution of prostate carcinoma" CANCER, vol. 85, mai 1999 (1999-05), pages 2017-2022, XP001025560
- JENKINS R B ET AL.: "A molecular cytogenetic analysis of 7q31 in prostate cancer" CANCER RESEARCH, vol. 58, 1998, pages 759-766, XP002168427 cité dans la demande
- MACOSKA J A ET AL.: "8p22 loss concurrent wit 8c gain is associated with poor outcome in prostate cancer" UROLOGY, vol. 55, mai 2000 (2000-05), pages 776-782, XP001001408
- PERINCHERY G ET AL.: "Deletion of Y-chromosome specific genes in human prostate cancer " THE JOURNAL OF UROLOGY, vol. 163, avril 2000 (2000-04), pages 1339-1342, XP001025513 cité dans la demande

## Description

La présente invention a trait à des procédés de diagnostic *in vitro* et/ou de pronostic du cancer de la prostate à partir d'un échantillon de cellules, contenues dans les sécrétions, d'origine prostatique récoltées de manière non invasive dans les urines après le massage de la prostate ou de manière peu invasive via une aiguille à biopsie, ou cytoponction. Elle porte aussi sur un kit de diagnostic permettant une mise en oeuvre simple du procédé.

Le cancer de la prostate est l'un des cancers les plus répandus chez l'homme dans les pays industrialisés et son incidence augmente de manière importante ces dernières années. L'étiologie du cancer de la prostate est complexe et englobe à la fois des facteurs génétiques (prédisposition familiale, origine ethnique) et exogènes (carcinogènes) variables avec l'environnement socio-économique, les habitudes alimentaires, la situation géographique et la pollution.

Le diagnostic précoce de cette maladie, qui est la première cause de mortalité par cancer chez l'homme de plus de 50 ans, est actuellement le seul moyen de pouvoir proposer un traitement capable d'obtenir une guérison.

Le pronostic dépend du stade de la maladie au moment du diagnostic : en effet, seul un cancer de la prostate localisé peut être traité de manière efficace par prostatectomie ou radiothérapie. Cependant, une grande proportion de patients est à un stade avancé de la maladie au moment du diagnostic : environ 28% des patients ont une extension extra prostatique et 25% ont des métastases (majoritairement ganglionnaires et osseuses).

Le cancer de la prostate à un stade précoce, donc curable ne donne pas de symptôme et ne peut être suspecté que par des méthodes de dépistage dont la plus utilisée actuellement est le dosage du PSA (prostate specific antigen) sanguin. La suspicion de cancer par une méthode de dépistage nécessite une confirmation anatomo-pathologique sur des prélèvements de tissu obtenus par biopsies de la prostate. Le PSA est une protéase de la famille des kallicréines, sécrété à l'état physiologique par les cellules épithéliales luminales (cellules épithéliales différentiée et androgénodépendantes) des glandes prostatiques. Le taux de PSA dans le sang peut s'élever au cours de différentes pathologies de la prostate (infections ou inflammations prostatiques (prostatites), traumatismes de la prostate, cancers de la prostates développées au dépend des cellules épithéliales (adénocarcinomes prostatiques) de la prostate, hyperplasie bénigne de la prostate) et servir de marqueurs pour ses pathologies. Le fait que le PSA ne soit pas un marqueur spécifique des adénocarcinomes prostatiques fait qu'il a une faible valeur prédictive de biopsies positives dans le diagnostique précoce des cancers de la prostate (moins de 20% de VPP pour des valeurs du taux de PSA < à 4ng/ml,; moins de 40% pour PSA <10ng/ml). La valeur seuil habituellement reconnue pour indiquer des biopsies prostatiques (suspicion de cancer) est 4ng/ml ( l'utilisation de ce seuil revient à faire des biopsies de la prostate chez environ 10% des hommes de plus de 50 ans). Cependant, avec ce seuil, 40% des hommes supposés présenter une tumeur localisée auront déjà développé une tumeur extra-prostatique (1) et 20% des cancers sont méconnus. L'abaissement du seuil à 2ng/ml permet de diagnostiquer plus de cancer et plus de cancers curables car encore limités à la prostate, cependant et en raison de la mauvaise spécificité du PSA cela revient à faire près des biopsies prostatiques à près de 30% des hommes de plus de 50 ans.

La méthode de confirmation diagnostique classiquement consiste en la réalisation de biopsies (entre 6 à 10) de la prostate réalisée par voie transrectale , parfois par voie périnéale), les biopsies sont dirigées sur la prostate sous le contrôle d'une échographique endorectale ou du toucher digital. Les biopsies sont pratiquée après un dosage sanguin de l'antigène prostatique spécifique (PSA : Prostate Specific Antigen) dans le sang. De plus, les biopsies prostatiques sont invasives et douloureuses (nécessitent parfois une anesthésie générale) pour le patient et susceptible d'engendrer des complications infections et saignements dans prés de 10% des cas. Le risque de méconnaître un cancer de la prostate par les biopsies n'est pas négligeable prés de entre 20 et 30%,. En effet dans la majorité des cas le cancer n'est pas palpable, ni visualisable par imagerie au sein de la glande prostatique, si bien que les biopsies sont faites au hasard dans la prostate. Le risque de méconnaître un cancer par biopsies sera d'autant plus grand que la prostate est volumineuse (petit volume de cancer dans un important volume prostatique). Ces limites à la sensibilité des biopsies prostatiques font souvent répéter les biopsies. Pour accroître la valeur prédictive du PSA et éviter de pratiquer beaucoup de biopsies inutiles, plusieurs modalités pour pondérer le taux de PSA ont été proposés (PSA rapporté à l'âge, PSA rapporté au volume de la prostate (Densité du PSA), Cinétique du PSA dans le temps). Ces méthodes de pondérations sont cependant inférieures en terme de valeurs prédictives de biopsie positive à l'utilisation du rapport des formes libres et complexées du PSA dans le sang. En effet le PSA dit total dosé dans le sang est sous un forme libre et sous une formes complexés à des protéines telles que l'alphachymotrypsine. Plusieurs méthodes diagnostiques basées sur la reconnaissances d'épitopes du PSA masqués ou non par les protéines qui le complexent, permettent de réalisé soit un dosage du PSAtotal, soit du PSA libre, soit du PSA complexé. Pour des raisons encore inconnues, il s'avère que dans le cancer de la prostate, par rapport aux autres pathologies prostatiques (comme l'hypertrophie bénigne), le rapport entre le PSA libre et le PSA total s'abaisse (d'autres ratio utilisant le taux de PSA total, ou complexé ou libre peuvent également être utilisé, il ont la même signification). L'utilisation du ratio PSA libre/total a permis d'apporter une légère amélioration de la spécificité dans le diagnostic précoce du cancer de la prostate (2). Cependant les seuils proposés pour indiquer les biopsies prostatiques ne sont pas formellement définis. Un taux <15% est très fortement évocateur de cancer, un taux entre 15 et 25% suspect et rassurant s'il est > à 25%.

A coté du PSA d'autres marqueurs circulants (comme les nuclear Matrix protein NMP) sont en cours d'évaluation pour le diagnostic du cancer de la prostate. Par ailleurs, il a été proposé une méthode non-invasive de détection moléculaire de marqueurs (télomérase, GSTP1) spécifiques des cellules cancéreuses et/ou saines de la prostate à partir de cellules récoltées dans des éjaculas ou après lavage des urètres chez des patients. Cependant, les résultats obtenus dans cette étude ne permettent pas de discriminer les cellules tumorales des cellules normales (3). Par conséquent, il existe toujours un réel besoin d'apporter de nouveaux procédés de détection précoce du cancer de la prostate qui améliorent la sensibilité, la spécificité et la prédictibilité et qui utilisent de préférence des moyens non-invasifs.

L'accumulation d'altérations génétiques caractérise la transformation d'une cellule normale vers la cellule cancéreuse chez l'homme. En particulier, une cellule deviendrait cancéreuse par l'activation d'oncogènes et l'inactivation fonctionnelle de gènes suppresseurs de tumeurs. Selon le type histopathologique de cancers, les régions chromosomiques affectées par des délétions et ce de façon récurrente sont plus ou moins caractéristique. Ceci a déjà été utilisé, a des fins diagnostic, pour détecter des cellules tumorales de cancers de la vessie, du colon ou du poumon dans des fluides biologiques. Ces altérations chromosomiques sont détectées par la mise en évidence soit d'un déséquilibre allélique ou perte d'hétérozygotie (Loss of heterozygosity, LOH) soit d'une délétion homozygote, ces deux phénomènes étant regroupés dans la suite de la description et dans les revendications sous l'expression générale "perte d'allèles". Des pertes d'allèles associées à des tumeurs du cancer de la prostate ont été détectées de façon récurrente sur les bras chromosomiques 6p, 6q, 7q, 8p, 9p, 10q, 12p, 13q, 16q, 17q,18q et Yq. L'essentiel des études sur les pertes d'allèles (hétérozygotes ou homozygotes) a porté sur la recherche de la localisation chromosomique précise de ces altérations en vue de l'éventuelle identification de gènes suppresseurs de tumeurs. Ces études ont aussi permis de caractériser la fréquence de . détection de ces anomalies dans des tumeurs du cancer de la prostate.

L'invention résulte de la recherche d'altérations chromosomiques par la détection de LOH ou de délétion homozygote parmi plusieurs régions caractérisées (hot spot) à partir de cellules cancéreuses d'origine prostatique obtenues de manière non invasive ou peu invasive. Les résultats obtenus montrent que cette recherche peut être utilisée de façon avantageuse comme moyen de diagnostic du cancer de la prostate chez des patients à risque. En pratique, cette recherche est conduite postérieurement à la détection dans le sang d'un taux de PSA élevé et d'un ratio PSA libre/total anormal, le diagnostic ou pronostic obtenu par le procédé de l'invention étant ensuite, en cas de positivité, confirmé par le biais d'une biopsie. En particulier, il a pu être montré que la détection de pertes d'allèles parmi au moins une région chromosomique choisie dans le groupe comprenant les régions 6p21-31, 6q21, 6q25.3, 7q31, 8p22, 9p21, 10q23-25, 12p13, 13q14, 16q24.3, 16q23.2, 17q21.2, 18q21 et Yq11-12, sur un échantillon de cellules d'origine prostatique, en association avec la caractérisation desdites cellules par l'analyse de plusieurs marqueurs spécifiques de cellules de la prostate, permet d'augmenter simultanément la sensibilité, la spécificité et les valeurs prédictives positives et négatives du diagnostic du cancer de la prostate. En conséquence, le procédé de diagnostic objet de la présente invention permet, en cas de résultat négatif, de ne pas effectuer de biopsie, laquelle était indispensable avec les diagnostics connus de l'état de la technique.

Ainsi, l'invention porte sur un procédé de diagnostic *in vitro* et/ou de pronostic du cancer de la prostate à partir d'un échantillon de cellules d'origine prostatique isolées d'un patient tel que défini dans les revendications.

En d'autres termes, l'invention consiste à analyser dans une première étape la qualité du prélèvement, c'est à dire sa richesse en cellules d'origine prostatique de sorte à permettre d'effectuer, dans une seconde étape, une recherche d'altérations chromosomiques dont les résultats soient significatifs et spécifiques des cellules cancéreuses d'origine prostatique. Le procédé est conduit sur des cellules d'origine prostatiques, c'est-à-dire tant sur des cellules prostatiques que sur des cellules métastasées, en particulier sur des cellules cancéreuses d'origine prostatique ayant envahi les ganglions lymphatiques et les os et ce, de manière à permettre de vérifier une éventuelles proliférations des cellules cancéreuses d'origine prostatiques vers d'autres organes.

La récolte des cellules d'origine prostatique peut être réalisée de manière peu invasive à l'aide d'une aiguille à biopsie. Dans une forme préférée de mise en oeuvre du procédé, les cellules sont récoltées dans les urines après massage de la prostate par voie rectale, de sorte que l'échantillon de cellules prostatiques récupéré résulte, au sens de l'invention, d'un mélange de cellules prostatiques et d'urine. Le massage permet en outre d'augmenter le nombre de cellules d'origine prostatique présentes dans les urines. Cette méthode a l'avantage d'être non invasive et convient particulièrement à la répétition dans le temps du diagnostic. Il est donc possible, pour procéder à l'analyse ultérieure de la qualité du prélèvement, de travailler soit sur l'ARN des cellules d'origine prostatiques soit directement sur l'urine. Ainsi et selon une première caractéristique du procédé, l'extraction des ADN et ARN des cellules prostatiques peut être effectué après centrifugation de l'échantillon et récupération du culot cellulaire ou un autre procédé de tri cellulaire permettant de récupérer spécifiquement les cellules d'origine prostatique. La collecte des cellules d'origine prostatique lorsqu'il s'agit de cellules métastasées est réalisée par prélèvement sanguin ou prélèvement des ganglions lymphatiques lors d'une prostatectomie radicale.

Une double-extraction ADN et ARN est ensuite réalisée de préférence simultanément à partir des échantillons cellulaires récoltés et triés le cas échéant. Des protocoles de double-extraction sont décrits dans l'état de la technique et sont par exemple décrits par Chomczynski & Sacchi (14). L'objectif est de permettre l'extraction simultanée des ARN et des ADN à partir d'un même échantillon cellulaire.

Un des avantages du procédé de l'invention est de fournir comme déjà dit, une étape d'analyse tant qualitative que quantitative des cellules présentes dans l'échantillon. En effet, selon une première forme de réalisation du procédé de l'invention, différents marqueurs spécifiques des cellules d'origine prostatique sont détectés à partir des ARN extraits. On entend par « marqueur spécifique des cellules d'origine prostatique», toute molécule ou macromolécule présente en quantité, structure ou conformation inhabituelle dans ou à la surface des cellules d'origine prostatique par rapport aux cellules non prostatiques. Dans le procédé de l'invention, une association de marqueurs est utilisée de manière à caractériser l'origine des cellules et certains types cellulaires et leur niveau de différenciation (cellules endothéliales, cellule épithéliales, cellules stromales et cellules basales épithéliales). La caractérisation des cellules par l'utilisation d'une association de marqueurs permet également d'augmenter la sensibilité du diagnostic compte tenu de la possibilité de perte de certains marqueurs prostatiques dans les tumeurs évoluées. Des marqueurs préférés sont les marqueurs PSA (prostate specific antigen), PSMA (prostate specific membrane antigen), PAP (prostate acid phosphatase), la cytokératine 5 (KRT5), la cytokératine 18, (KRT18), la vimentine et le marqueur CD34. Le type ou l'état cellulaire est ainsi déterminé par la détection et/ou la quantification des différents marqueurs utilisés. La détection comprend généralement une étape de transcription inverse des ARNm en ADNc puis une étape d'amplification quantitative permettant de quantifier les marqueurs spécifiques dans chaque échantillon d'ADNc amplifié.

Toute technique permettant l'amplification spécifique d'un acide nucléique donné peut être utilisée dans le procédé de l'invention. Citons à titre d'exemple la méthode d'amplification PCR (Polymerase Chain Reaction) ou les méthodes d'amplification isotherme telles que la TMA (transcription mediated amplification), la NASBA (nucleic acid sequence based amplification), la 3SR (self sustained sequence réplication) ou l'amplification par déplacement de brin (strand displacement amplification). De manière préférée, des amorces choisies pour l'amplification des marqueurs spécifiques PSA, PSMA, PAP, KRT5, KRT18, vimentine et CD34 sont définies par les séquences nucléotidiques présentées dans le tableau 1 ci-dessous.

**Tableau 1   Exemples de séquences nucléotidiques d'amorces pouvant être utilisées pour l'amplification des marqueurs cellulaires**

| KRT 18 | 5'-TGA GAC GTA CAG TCC AGT CCT-3' |
|--------|-----------------------------------|
|        | 5'-GCT CCA TCT GTA GGG CGT AG-3'  |
| PAP    | 5'-CAT CTG GAA TCC TAT CCT ACT CTG-3' |
|        | 5'-AGT TCT TGA AAA CGA GGG CA-3'  |

| PSA       | 5'-ACC AGA GGA GTT CTT GAC CCC AAA-3' |
|-----------|---------------------------------------|
|           | 5'-CCC CAG AAT CAC CCG AGC AG-3'      |
| CD34      | 5'-GTG TCT CAA CAT GGC AAT GAG G-3'   |
|           | 5'-ACA GAG GTC TGT GAC TGG ACA GAA-3' |
| KRT5      | 5'-CCG TGC CGC AGT TCT ATA TTC-3'     |
|           | 5'-GTG GGG ATT CTG TTT TGA TGA TT-3'  |
| VIMENTINE | 5'-CCT TGA ACG CAA AGT GGA ATC-3'     |
|           | 5'-GAC ATG CTG TTC CTG AAT CTG AG-3'  |
| RPLP0     | 5'-GGC GAC CTG GAA GTC CAA CT-3'      |
|           | 5'-CCA TCA GCA CCA CAG CCT TC-3'      |

Différentes méthodes de quantification des acides nucléiques sont proposées dans l'état de la technique. Parmi les techniques possibles, on peut citer la quantification par électrophorèse en gel d'agarose, mesure de la densité optique (DO), slot blot, ou Northern blot. Des méthodes de quantification préférées pour la quantification des produits amplifiés sont celles utilisant des intercalants fluorescents tel que le système SYBrGreen commercialisé par Roche ou des couples de groupements fluorophores/quenchers telles que le système TaqMan™ commercialisé par ABI®, le système AmpliSensor™ commercialisé par InGen ou encore le système Sunrise™ commercialisé par Oncore et Appligène®. Afin de pouvoir estimer la quantité relative de chaque transcrit amplifié et de tenir compte de la variabilité de la quantité d'ARN total extrait, un contrôle interne est utilisé. Le contrôle interne est un transcrit choisi pour son absence de rétro-pseudogènes contrairement aux contrôles classiquement utilisés tels que la GAPDH ou la β-actine. Un tel contrôle interne peut être par exemple le transcrit du gène RPLPO codant pour une protéine ribosomale. L'expression relative (normalisée par rapport au contrôle interne) d'un marqueur donné, dans un échantillon, est également normalisée par rapport à l'échantillon présentant le plus faible taux d'expression : le calibrateur. Ceci permet ainsi de définir le niveau d'expression relatif de chaque marqueur par rapport au contrôle interne choisi et au calibrateur. Le calcul de la valeur est résumé comme suit:

$$\frac{\text{Marqueur}^{\text{échantillon}}/\text{Contrôle interne}^{\text{échantillon}}}{\text{Marqueur}^{\text{calibrateur}}/\text{contrôle interne}^{\text{calibrateur}}}$$

Plus généralement, toute calibration permettant une évaluation quantitative des transcrits, notamment celles qui éliminent les biais introduits lors des amplifications de transcrits de tailles différentes, peut être valablement utilisée dans cette étape du procédé de l'invention.

Dans une seconde forme de réalisation du procédé de l'invention, l'étape d'analyse de la qualité de l'échantillon est effectuée par dosage des antigènes spécifiques des cellules prostatiques directement dans l'urine contenue dans l'échantillon de cellules d'origine prostatiques. On peut ainsi doser directement au moins une protéine choisie dans le groupe comprenant PSA, PSMA, PAP, prostase, par toute technique connue de l'homme du métier en particulier, Western blot, immunofluorescence, technique ELISA, et micropuces à protéines.

Après la caractérisation de l'échantillon de cellules à partir des ARN totaux extraits conformément aux étapes a) et b) du procédé ou dosage d'antigènes dans l'urine, l'ADN de chaque échantillon présentant un taux suffisant de cellules d'origine prostatique est analysé pour la recherche d'altérations chromosomiques détectées par une perte d'allèles sur au moins une région chromosomique fréquemment associée au cancer de la prostate.

Tout type de marqueurs de polymorphisme peut être utilisé dans le procédé de l'invention pour permettre une détection des pertes d'allèles. On peut citer en plus des marqueurs microsatellites, par exemple les marqueurs de polymorphisme de longueur de fragments de restriction (RFLP) ou encore les marqueurs SNP (Single Nucleotide Polymorphism).

De manière préférée, la détection d'altérations chromosomiques est réalisée par l'analyse de marqueurs microsatellites de polymorphisme localisés au niveau des régions altérées. Un mode de réalisation de l'invention consiste à amplifier la région porteuse des marqueurs de polymorphisme et à quantifier les produits amplifiés issus des deux allèles.

Les marqueurs sont alors choisis de manière à être facilement amplifiables pour leur quantification.

La méthode d'amplification est choisie et optimisée de sorte que les produits générés soient représentatifs en quantité et en qualité de la matrice d'amplification. Une amplification de l'ADN extrait des cellules d'origine prostatique est réalisée en parallèle d'une amplification de l'ADN constitutionnel extrait de cellules normales prélevées sur le même patient et représentant les allèles non délétés. Une co-amplification avec des marqueurs situés dans des régions non altérées permet également de constituer un standard interne afin de valider les pertes d'allèles observées dans les régions d'intérêts. Seuls sont pris en compte pour le diagnostic les échantillons d'acides nucléiques dits « informatifs », c'est à dire, les échantillons pour lesquels les marqueurs présentent deux allèles différents pour une région donnée. De manière préférée, afin d'augmenter le nombre d'échantillons informatifs, au moins deux marqueurs par région analysée sont choisis.

Dans le procédé de l'invention, une perte d'hétérozygotie est détectée lorsque l'intensité relative des deux allèles est significativement différente de l'intensité relative des deux allèles issus de l'analyse d'un échantillon d'ADN contrôle extrait par exemple de lymphocytes prélevés du sang du patient et traités en parallèle.

De manière préférée, une perte d'hétérozygotie est détectée lorsque le rapport entre les intensités relatives des deux allèles de l'ADN de cellules prostatiques tumorales et l'intensité relative des deux allèles des lymphocytes est en dehors de la fourchette 0.76-1.3. Les intensités de détection des deux allèles peuvent être mesurées selon les techniques classiques ou automatisée de quantification des acides nucléiques de type slot blot, ou électrophorèse en gel de polyacrylamide de produits radiomarqués, technologies de quantification de type TaqMan® ou avantageusement aujourd'hui par des biopuces à ADN ou des microarrays (1 5). Le principe de ces techniques repose sur l'hybridation d'un ou plusieurs acides nucléiques cibles avec un ou plusieurs acides nucléiques « sondes » spécifiques marqués, la quantité de « sondes » hybridées étant théoriquement proportionnelle à la quantité de «cibles» présentes sur le support d'hybridation. La quantification des allèles peut aussi être réalisée par les techniques d'amplification et de marquage à l'aide d'amorces portant un fluorophore puis séparation sur gel sur séquenceur tel que celui commercialisé par Perkin Elmer modèle 377, et quantification à l'aide par exemple du logiciel d'analyse Genescan© 672 Fragment Analysis (Applied Biosystem) qui calcule la taille et la surface des pics.

Les régions chromosomiques analysées pour la recherche d'altérations chromosomiques sont choisies parmi les régions où une perte d'allèles est fréquemment détectée sur des cellules tumorales prostatiques et pour lesquelles aucune interférence ou hybridation croisée ne peut être attendue ni observée lors des étapes d'amplification ou d'hybridation (étapes b) et c) du procédé. L'analyse d'au moins une région chromosomique choisie dans le groupe comprenant les régions 6p21-31, 6q21, 6q25.3, 7q31, 8p22, 9p21, 10q23-25, 12p13, 13q14, 16q23.2, 16q24.3, 17q21.2, 18q21, et Yq11-12 permet selon le procédé, un diagnostic dont la sensibilité, la spécificité et la prédictibilité sont améliorées par rapport aux diagnostics actuellement en routine, qui présentent l'inconvénient d'être traumatiques.

Avantageusement, les pertes d'allèles sont recherchées sur les 3 régions chromosomiques suivantes : 7q31, 8p22, 13q14 ; alternativement sur les 4 régions chromosomiques suivantes : 7q31, 8p22, 13q14, 16q23.2 , de préférence sur les 6 régions chromosomiques suivantes : 7q31, 8p22, 12p13, 13q14, 16q23.2, et 18q21 ; plus le nombre de régions étant important et plus les résultats étant significatifs.

Dans le procédé de l'invention, une délétion homozygote est détectée par la quantification du nombre d'allèles présents par des techniques de mesure telles que le système TaqMan en utilisant comme marqueur, en particulier le marqueur D12S89 localisé entre D12S77 et D12S358.

Les régions chromosomiques analysées pour la recherche d'altérations chromosomiques au niveau des cellules tumorales d'origine prostatique sont choisies parmi les régions où des délétions d'allèles sont fréquemment détectées.

Dans un mode de réalisation du procédé, des amorces sont choisies de manière à réaliser des amplifications PCR multiplex. On entend par PCR multiplex, l'amplification de plusieurs séquences cibles (deux au moins) dans un même tube d'amplification. Des amorces spécialement adaptées pour l'amplification des marqueurs microsatellites situés aux régions 6p21-31, 6q21, 6q25.3, 7q31, 8p22, 9p21, 10q23-25, 12p13, 13q14, 16q23.2, 16q24.3, 17q21.2 et 18q21 sont celles dont la séquence nucléotidique est définie dans Dib et al (17) et Tomforhrde et al (19). Les marqueurs utilisés pour les régions Yq11-12 sont donnés par des gènes référencés par Perinchery et al. (18). Les marqueurs utilisés pour chaque région chromosomique sont reproduits dans le tableau ci-après :

| REGION CHROMOSOMIQUE | MARQUEUR |
|---|---|
| 6p21-31 | D6S1645, D6S1611, D6S291 |
| 6q21 | D6S404, D6S302 |
| 6q25.3 | D6S3398, D6S440, D6S420 |
| 7q31 | D7S523, D7S480, D7S522 |
| 8p22 | D8S1786, D8S133, D8S261 |
| 9p21 | D9S 1846, D9S171, D9S169 |
| 10q23-25 | D10S1765, D10S2491, D10S541, D10S1237 D10S587, D10S1223 |
| 12p13 | D12S77, D12S358, D12S89 |
| 13q14 | D13S272, D13S153, D13S284 |
| 16q23.2 | D16S507, D16S518 |
| 16q24.3 | D16S3098, D16S413, D10S520 |
| 17q21.2 | D17S932, D17S800, D17S855 |
| 18q21 | D18S39, D18S46 |
| Yq11-12 | BPY1, SMCY, RBM1, BPY2 |

Dans une forme de réalisation avantageuse du procédé de l'invention, on fait suivre l'étape de recherche d'altération chromosomique, d'une étape de détection puis de quantification du marqueur de la neuropiline (NRP1) dans l'échantillon, dont on sait qu'il est fréquemment surexprimé dans les cellules cancéreuses d'origine prostatiques.

Les techniques de détection et d'amplification quantitatives sont identiques à celles mises en oeuvre pour les marqueurs PSA, PSMA, PAP, prostase lors de l'étude qualitative du prélèvement de cellules d'origine prostatique.

En lieu et place de la neuropiline, on peut également détecter toute protéine impliquée dans la voie de l'angiogénèse ou de l'apoptose dans l'échantillon.

De manière préférée, des amorces choisies pour l'amplification des marqueurs spécifiques de la neuropiline sont choisies parmi les séquences de nucléotides suivantes : 5'-AGT GTC TTG CAG AGC AGT GTC TCA- 3' et 5'-TGG TGC TAT ACT GGG AAG AAG CTG T- 3'.

Le procédé de l'invention permet ainsi d'obtenir en particulier une caractérisation des cellules analysées, la quantité relative de PSA (normalisée avec un contrôle interne), le rapport PSA libre/total, l'analyse de la présence d'altérations chromosomiques sur au moins une région chromosomique et enfin, éventuellement la quantité de neuropiline dans l'échantillon. Les résultats obtenus dans la partie expérimentale présentent des exemples de mise en oeuvre du procédé montrent de manière surprenante que la détection d'au moins une perte d'allèle sur trois, quatre et six régions chromosomiques analysées permet d'améliorer la qualité du diagnostic du cancer de la prostate de manière significative en comparaison avec le diagnostic classiquement utilisé, consistant en l'analyse du ratio PSA libre/total considéré comme significatif d'un mauvais pronostic lorsque celui-ci est inférieur à 25%. Plus particulièrement, il est montré qu'un test de diagnostic consistant en la détection d'au moins une perte d'allèles parmi les quatre régions analysées suivantes : 7q31, 8p22, 13q14 et 16q23.2 permet d'augmenter les valeurs de sensibilité, de spécificité et les valeurs prédictives positives et négatives de respectivement 8, 15, 20 et 17 points par rapport aux valeurs obtenues avec la détection du ratio PSA libre/total. Par conséquent, la mise en oeuvre du procédé selon l'invention fournit un nouveau test in vitro et non ou peu invasif qui contribue avec l'analyse du ratio PSA libre/total à améliorer le diagnostic du cancer de la prostate et fournit un pronostic en vue de la décision d'une intervention ultérieure, telle que la réalisation d'une biopsie de la prostate.

De plus, la mise en oeuvre du procédé chez un nombre élevé de patients ainsi que le suivi clinique de la réponse aux traitements thérapeutiques ultérieurs réalisés pour les patients atteints d'un cancer permet la constitution d'une base de données associant les caractéristiques phénotypiques des cellules tumorales (obtenues à l'étape b) du procédé, les caractéristiques génotypiques des cellules tumorales (obtenues à l'étape c) du procédé et les données cliniques portant sur la réponse aux traitements thérapeutiques ultérieurs.

Un médecin peut alors par consultation d'une base de données, affiner le diagnostic et le pronostic réalisés sur l'un de ses patients. Cela lui permet ainsi de proposer un traitement thérapeutique en fonction des caractéristiques phénotypiques et génotypiques des cellules tumorales de son patient, et du tableau clinique de ce dernier. L'itération du procédé et l'enregistrement des données acquises sur chacun des patients permet d'enrichir au cours du temps la base de données et ainsi augmenter la fiabilité du diagnostic et/ou du pronostic établi par le médecin, et par là même, la pertinence du traitement proposé.

Un autre objet de l'invention est un kit pour le diagnostic ou/et le pronostic in vitro du cancer de la prostate mettant en oeuvre les procédés décrits précédemment. Un tel kit comprend au moins un de chacun des éléments suivants :

- des amorces spécifiques permettant l'amplification des ARN correspondant aux marqueurs spécifiques des cellules d'origine prostatiques et/ou un réactif de dosage des antigènes spécifiques des cellules prostatiques;
- des amorces spécifiques permettant l'amplification spécifique de séquences porteuses des marqueurs de polymorphisme,
- les tampons et enzymes nécessaires aux réactions d'amplification, de marquage et d'hybridation
- le cas échéant, les cibles pour l'hybridation spécifique des produits amplifiés et leur quantification, lesdites cibles pouvant être fixées sur un support.-

Selon une forme préférée de réalisation de l'invention, le kit contient des amorces choisies pour l'amplification spécifique des séquences porteuses des marqueurs de polymorphismes situées au niveau des régions 6p21-31, 6q21, 6q25.3, 7q31, 8p22, 9p21, 10q23-25, 12p13, 13q14, 16q24.3, 16q23.2, 17q21.2, 18q21 et Yq11-12 lesquelles présentent des LOH ou des délétions homozygotes. Des marqueurs de polymorphismes peuvent être par exemple des marqueurs microsatellites, des marqueurs SNP ou RFLP. Des exemples d'amorces spécialement adaptées pour l'amplification de marqueurs microsatellites situés au niveau de l'ensemble de ces régions sont celles dont la séquence nucléotidique est définie dans Dib et al (17), Tomforhrde et al (19), et Perinchery et al. (18).

Dans une forme de réalisation avantageuse, le kit contient des amorces permettant l'amplification spécifique des séquences porteuses des marqueurs de polymorphismes situées au niveau des trois régions 7q31, Sp22 et 13q14.

Selon une autre forme de réalisation, le kit contient des amorces permettant l'amplification spécifique des séquences porteuses des marqueurs de polymorphismes situées au niveau des quatre régions 7q31, 8p22, 13q14 et 16q23.2.

De préférence, le kit contient des amorces permettant l'amplification spécifique des séquences porteuses des marqueurs de polymorphismes situées au niveau des six régions 7q31, 8p22, 12p13, 13q14, 16q23.2, et 18q21.

Des amorces préférées pour l'amplification des ARN correspondant aux marqueurs spécifiques des cellules prostatiques et cancéreuses sont celles permettant l'amplification des gènes PSA, PSMA, PAP, KRT 18, la vimentine, KRT 5 et CD34. Par exemple, de telles amorces sont celles dont la séquence nucléotidique est définie dans le tableau 1 ci-dessus.

Le kit contient en outre un réactif permettant de doser au moins un antigène spécifique présent dans l'urine contenue dans l'échantillon choisi parmi PSA, PSMA, PAP ou prostase. La technique utilisée est en pratique la technique ELISA ou toute technique équivalente comme le western blot, l'immunofluorescence, ou les puces à protéines.

De manière optionnelle, les cibles pour l'hybridation spécifique des produits amplifiés sont comprises dans le kit, lesdites cibles pouvant être fixées sur un support. L'hybridation entre les produits d'amplification et les cibles spécifiques des régions chromosomiques étudiées peut être réalisée dans des conditions classiques, telles que celles décrites par Sambrook et al. (20).

La fixation stable des acides nucléiques cibles sur un support permet de réaliser une analyse quantitative et qualitative simple et rapide des produits amplifiés par hybridation spécifique des échantillons d'ADN amplifiés et préalablement marqués, avec les ADN cibles présents sur le support, puis par quantification du signal d'intensité d'hybridation. De tels supports sont des membranes de nylon ou de nitrocellulose classiquement utilisées en biologie moléculaire pour la quantification des acides nucléiques sur lesquelles ont été transférés ou déposés les ADN cibles. Ces supports peuvent aussi être des supports solides de types puces à ADN ou microarrays (16).

Dans une forme de réalisation préférée, le kit contient en outre des amorces pour l'amplification du marqueur de la neuropiline (NRP1), dont les séquences nucléotidiques sont choisies parmi les séquence suivantes : : 5'-AGT GTC TTG CAG AGC AGT GTC TCA- 3' et 5'-TGG TGC TAT ACT GGG AAG AAG CTG T-3'.

L'invention porte aussi sur l'utilisation d'un kit tel que décrit précédemment pour inclure les résultats obtenus dans une base de données constituée des données phénotypiques et génotypiques de patients atteints de cancer de la prostate ainsi que de leurs tableaux cliniques et de leur évolution en fonction des traitements et identifier le traitement thérapeutique optimal à proposer au patient.

Les exemples qui suivent illustrent des modes de réalisation de l'invention sans en limiter la portée des revendications. Ils présentent par ailleurs une étude comparative du diagnostic par le procédé de l'invention avec le diagnostic par la mesure du ratio PSA libre/total.

### Exemple 1 : détection de la perte d'hétérozygotie sur quatre régions chromosomiques indépendantes chez 32 patients

Afin de déterminer l'efficacité d'un diagnostic du cancer de la prostate par la détection d'au moins une perte d'hétérozygotie sur 4 régions indépendantes, une étude a été menée sur 32 patients âgés de 55 à 81 ans.

### A : Matériels et méthodes

### A1 : Prélèvement des cellules

Les patients qui ont été choisis pour le prélèvement des cellules ont un niveau de PSA resté supérieur à 4 ng/ml pendant 3 à 6 mois après une première analyse négative de la prostate par biopsie.

Après massage par voie rectale de la prostate, les urines des 32 patients ont été collectées puis centrifugées pour récolter les cellules présentes. En parallèle, un prélèvement sanguin a été réalisé.

### A2 Extraction des ADN et ARN des cellules

L'ADN des lymphocytes sanguins prélevés a été extrait. L'ADN et l'ARN ont été extraits de manière simultanée à partir des cellules récoltées dans les urines selon le protocole suivant

*Traitement des culots urinaires*

Le culot est ensuite mis à décongeler dans la glace. Deux fois 250 µl de solution D sont ajoutés au culot, puis le culot est transféré dans un tube Eppendorf. Cinquante microlitres d'acétate de sodium 2M pH4 (concentration finale de 0.2 M) sont ajoutés à l'échantillon, puis l'échantillon est agité. On ajoute alors 550 µl (volume à volume) de phénol acide pH 4,3 et 200 µl (1/5 du volume), de chloroforme/alcool isoamylique (c.i.a. 49/1). L'échantillon est agité vigoureusement pendant 10 secondes, puis mis à reposer dans la glace pendant 15 minutes. L'échantillon est ensuite centrifugé 15 minutes à 13 000 g à 4°C. La phase aqueuse (supérieure) contient les acides nucléiques. La moitié supérieure du surnageant est réservée à l'extraction des ARN alors que l'ADN est récupéré à partir de la moitié inférieure du surnageant, interface comprise.

*Extraction des ARN:*

Un volume d'isopropanol à -20°C est ajouté à la partie récupérée pour l'ARN et l'échantillon est placé à -20°C de 1 à 16 heures. L'échantillon est ensuite centrifugé pendant 30 minutes à 12 500 g à 4°C. Le surnageant est éliminé, puis le culot est repris dans 300 µl de solution D et mélangé. Un volume d'isopropanol à -20°C est ajouté et l'échantillon est placé à -20°C de 1 à 16 heures. L'échantillon est centrifugé 30 minutes à 13 000 g à 4°C. Le surnageant est ensuite éliminé puis le culot est lavé à l'éthanol 70% à froid. L'échantillon est ensuite centrifugé 10 minutes à 13 000 g. Le surnageant est éliminé et le culot est séché. L'ARN est repris dans 20 µl d'eau DEPC (distillée).

*Extraction de l'ADN:*

Sur la partie basse de la phase supérieure (interphase comprise), on ajoute un volume de phénol/chloroforme/ alcool isoamylique (p.c.i. 25 :24 :1). Après agitation, l'échantillon est centrifugé 5 minutes à 13 000g à 4°C. La phase aqueuse est récupérée et l'extraction au p.c.i. est renouvelée une fois. La phase aqueuse est ensuite récupérée. Un volume de chloroforme/alcool isoamylique (c.i.a. 24 :1) est ajouté, puis l'échantillon est centrifugé pendant 5 minutes à 13 000g à 4°C. La phase aqueuse est récupérée et l'extraction au c.i.a. est renouvelée une fois. Après récupération de la phase aqueuse, un volume d'acétate d'ammonium 8 M (concentration finale de 2 M) est ajouté, et 2,2 volumes d'éthanol absolu à -20°C. L'échantillon est agité doucement et placé à -20°C de 1 à 16 heures. Il est ensuite centrifugé pendant 30 minutes à 13 000g à 4°C. Le surnageant est éliminé et le culot est rincé avec 500 µl d'éthanol 70% à froid. Après centrifugation et élimination du surnageant, le culot est séché. Le culot est ensuite resuspendu dans 20 µl de Tris 10mM-EDTA 1 mM pH 8.

A.3 Amplification. des ARN par RT-PCR

L'ARN total (1 µg) est reverse-transcrit dans un volume total de 20 µl contenant une solution tampon (500 µM DNTP, 3 mM $MgCl_2$, 75 mM KCl, 50 mM Tris-HCl pH 8.3), 10 unités de Rnasin Tm Ribonuclease inhibitor (Promega, Madison, WI), 10 mM de dithiothreitol, 50 unités de transcriptase inverse Superscript II Rnase H- (Gibco BRL, Gaithersburg, MD) et 1.5 µM d'hexamères aléatoires (Pharmacia, Uppsala, Sweden). Les échantillons sont incubés à 20°C pendant 10 minutes et à 42°C pendant 30 minutes, et la transcriptase inverse est inactivée par chauffage à 99°C. Les produits sont ensuite refroidis et conservés à 4°C.

La PCR est réalisée de manière quantitative selon la technologie TaqMan. La fluorescence générée par le clivage des sondes marquées est délectable à l'aide d'un détecteur de séquence (ABI Prism 7700 ou 7900 Sequence Detection System ; PE Applied Biosystems). Une courbe standard est réalisée à l'aide d'une série de dilution d'ADNc obtenus de tissus de prostate sains. Afin de tester le rendement de la PCR la courbe standard est composée de 5 points de dilutions (1 mg, 62.5 ng, 15.6 ng et 3.9 ng d'ADNc). Les transcrits RPLPO ont été quantifiés comme contrôle interne de chaque échantillon. Le transcrit PSA a par ailleurs été quantifié. Les amorces utilisées pour la PCR des transcrits RPLPO et PSA sont décrites dans le tableau 1 présenté plus haut.

A.4 Détection sur l'ADN des marqueurs microsatellites

Deux marqueurs microsatellites ont été utilisés par région analysée, les marqueurs D7S480, D7S522 pour la région 7q31, les marqueurs D8S133, D8S261 pour la région 8p22, les marqueurs D1 35153, D1 35284 pour la région 13q14 et les marqueurs D16S507 et D16S518 pour la région 16q23.2. Les séquences des amorces utilisées pour l'amplification de ces marqueurs microsatellites sont définies dans Dib et al. (17) et Tomforhrde et al. (19).

Les réactions de PCR sont réalisées sur un volume total de 15 µl contenant 50 ng d'ADN génomique, 3 pmole de chaque amorce (une marquée par flurorescence), 1 mM de chaque DNTP, 1.5 mM de MgCl2, 1 0 mM de Tris-HCl pH 8.3, 50 mM de KCl et 0.6 unités de Taq polymérase. Les conditions pour l'amplification sont : 94°C pendant 5 minutes, 12 cycles de 94°C pendant 15 secondes, 55°C pendant 15 secondes, 72°C pendant 30 secondes, suivie de 22 cycles de 89°C pendant 15 secondes, 55°C pendant 15 secondes et 72°C pendant 3 secondes. L'étape d'élongation finale à 72°C est prolongée à 10 minutes.

Un des deux oligonucléotides est marquée à la fluorescéine à son extrémité 5' et le séquenceur Applied Biosystems modèle 377 (PE) est utilisé pour détecter et quantifier les différents signaux. Un microlitre de produit de PCR est ajouté à 3,5 µl de formamide désionisé contenant 0,5 µl d'un marqueur de taille moléculaire (Genescan 500 ROX, Perkin Elmer). Le mélange est dénaturé à la chaleur et 2,5 µl sont déposés sur un gel de polyacrylamide 6% urée 8 M et mis à migrer pendant 2 à 3 heures dans le séquenceur. Les différents fragments sont quantifiés à l'aide du logiciel d'analyse Genescan& 672 Fragment Analysis (Perkin Elmer) qui calcule la taille et la surface des pics. Une perte d'hétérozygotie est considérée comme présente quand l'intensité relative des deux allèles de l'ADN analysé diffère de l'intensité relative des deux allèles de l'ADN des lymphocytes d'un facteur inférieur ou égal à 0,66 ou au moins égal à 1,5. Chaque analyse est réalisée au moins deux fois pour vérifier la reproductibilité de la détection de LOH (l'amplification PCR, la séparation par gel et la quantification sont répétées).

## B. Résultats

Le tableau 2 ci-dessous présente l'ensemble des données obtenues dans cette étude sur chacun des 32 patients. Plus précisément, il présente l'âge de chaque patient (Age), le numéro d'identification anonyme de chaque patient (DKPRO), les valeurs quantifiées et normalisées de PSA (PSA/RPLPO), le taux de PSA exprimé dans le sang (PSA), le ratio PSA libre/total (PSA LIT), l'évaluation des seuils (cut off) de 25% et 15% des ratio PSA libre/total (L/T<25% et L/T <15%) (test à valeur diagnostic), la détection d'au moins une perte d'hétérozygotie parmi les quatre régions analysées (test à valeur diagnostic selon l'invention), les résultats de l'analyse de la biopsie réalisée après le diagnostic (cancer de la prostate ou non) et enfin le niveau de différenciation de la tumeur suite à l'analyse histologique des biopsies positives.

**Tableau 2**: Présentation de l'ensemble des données obtenues sur l'étude clinique de 32 patients

| | Age | DKPRO | PSA/PO | PSA | PSA L/T | L/T>25% | L/T<15% | délété/non délété | Analyse biopsie | Différenciation |
|---|---|---|---|---|---|---|---|---|---|---|
| | 81 | 1428 | 944 | 11,7 | 29,9 | non | non | non | non | |
| | 58 | 1429 | 1263 | 10,8 | 15,7 | oui | non | délété | non | |
| | 73 | 1432 | 255 | 77 | 45 | non | non | délété | cancer de la prostate | Intermédiaire |
| | 61 | 1449 | 1213 | 27,5 | 10,5 | oui | oui | non | non | |
| | 68 | 1451 | 99 | 0,5 | 20 | oui | non | non | non | |
| | 61 | 1452 | 141 | 5,1 | 56,8 | non | non | délété | non | |
| | 70 | 1464 | 110 | 1,5 | 16,6 | oui | non | délété | cancer de la prostate | médiocre |
| | 65 | 1467 | 540 | 113 | 55 | non | non | délété | non | |
| | 66 | 1575 | 132 | 24,4 | 54,5 | non | non | non | non | |
| | 63 | 1578 | 732 | 16,4 | 18,2 | oui | non | noi | non | |
| | 58 | 1590 | 331 | 10,2 | 8,8 | oui | oui | non | non | |
| | 62 | 1592 | 1634 | 10,2 | 21,5 | oui | non | non | non | |
| | 70 | 159 | 478 | 24,8 | 43,5 | non | non | délété | non | |
| | 65 | 1596 | 423 | 6,5 | 35,3 | non | non | délété | non | |
| | 72 | 1618 | 696 | 30,1 | 26,5 | non | non | non | cancer de la prostate | mauvais |
| | 75 | 1628 | 1569 | 79,7 | 38,6 | non | non | non | cancer de la prostate | médiocre |
| | 55 | 1631 | 135 | 5,6 | 21,4 | oui | non | non | non | |
| | 71 | 1633 | 3120 | 59,9 | 7 | oui | oui | délété | cancer de la prostate | médiocre |
| | 67 | 1641 | 2246 | 7,4 | 16,2 | oui | non | délété | non | |
| | 79 | 1668 | 116 | 6 | 10,8 | oui | oui | délété | cancer de la prostate | bon |
| | 72 | 1669 | 221 | 13,5 | 12,9 | Cui | oui | délété | cancer de la prostate | mauvais |
| | 67 | 1685 | 1099 | 3,3 | 30,3 | non | non | non | non | |
| | 79 | 1687 | 830 | 6,6 | 40,9 | non | non | non | non | |
| | 68 | 1697 | 278 | 177 | 8,6 | oui | oui | délété | cancer de la prostate | mauvais |
| | 58 | 1705 | 1516 | 19,6 | 11,2 | oui | oui | non | non | |
| | 55 | 1708 | 166 | 104 | 57,3 | non | non | délété | non | |
| | 70 | 1772 | 334 | 3,5 | 28,5 | non | non | non | cancer de la prostate | bon |
| | 70 | 1826 | 271 | 6,3 | 39,68 | non | non | non | non | |
| | 80 | 2020 | 471 | 26,8 | 36,6 | non | non | délété | cancer de la prostate | |
| | 78 | 2025 | 5550 | 2 | 30 | non | non | délété | cancer de la prostate | médiocre |
| | 61 | 2040 | 279 | 2,4 | 25 | non | non | non | non | |
| | 62 | 2041 | 207 | 1,3 | 23 | oui | non | non | non | |
| Moyenne | 67,5 | | | 29,96 | 27,99313 | | | | | |
| Ecart-type | 7,37 | | | 40,55 | 15,24525 | | | | | |

Le nombre de perte d'hétérozygotie détectée pour chacune des quatre régions analysées est détaillé dans le tableau 3 ci-dessous ainsi que le nombre de cas par région chromosomique pour lesquels les résultats sont non informatifs compte tenu du fait que les cellules saines sont homozygotes pour le marqueur considéré.

Tableau 3

| Présentation des données obtenues sur la détection de perte d'hétérozygotie sur chacune des 4 régions indépendantes | | |
|---|---|---|
| **Régions chromosomiques analysées** | **Non Informatif** | **Anomalies chromosomiques (LOH)** |
| 7q31(D7S480; D7S522) | 3(8; 17) | 4(6;5) |
| 8p22(D8S133;D8S261) | 5(9; 12) | 1(3;2) |
| 13q14(D13S153; D13S284) | 0(4;3) | 1(6;4) |
| 16q23.2(D16S507; D16S518) | 7(8;9) | 2(2;3) |

Dans ce tableau, la colonne « non informatif » indique le nombre de patients testés dont la réponse est non informative. Les chiffres entre parenthèses indiquent le nombre de réponses non informatives obtenues pour les marqueurs pris individuellement. De la même façon, les résultats indiqués dans la colonne « LOH » indiquent l'existence d'une LOH pour les deux marqueurs simultanément, et entre parenthèses pour chacun des marqueurs pris individuellement.

Une biopsie positive a été considérée comme la référence standard pour le diagnostic d'un cancer de la prostate. Afin d'évaluer la valeur diagnostique du procédé selon l'invention, les paramètres de diagnostic fondés sur un test de détection de perte d'hétérozygotie (*positif* lorsqu'au moins une perte d'hétérozygotie est détectée) ont été comparés aux paramètres de diagnostic fondés sur un test de mesure du ratio PSA libre/total (positif lorsqu'il est inférieur à 25%), test classiquement utilisé aujourd'hui pour le diagnostic. Ces paramètres de sensibilité, de spécificité et de valeurs prédictives positives et négatives sont présentés dans le tableau 4.

Tableau 4: Comparaisons des paramètres de sensibilité, de spécificité et de valeurs prédictives selon le test réalisé pour le diagnostic

| Test diagnostic | Sensibilité | Spécificité | Valeur Prédictive | |
|---|---|---|---|---|
| | | | négative | positive |
| Détection d'au moins 1 LOH | 53% | 67% | 53% | 82% |
| PSA-libre/total < 25% | 45% | 52% | 33% | 65% |

Les résultats montrent que l'ensemble des paramètres est amélioré de manière significative. La sensibilité, la spécificité et les valeurs prédictives positives et négatives sont supérieures de respectivement 8, 15, 20 et 17 points avec le nouveau test diagnostic proposé et réalisable par la mise en oeuvre du procédé de l'invention. Par conséquent, ce nouveau test contribue avec l'analyse du ratio PSA libre/total à améliorer le diagnostic du cancer de la prostate et permet un pronostic, par exemple, pour la sélection des candidats à une biopsie de la prostate et un suivi de l'effet d'un traitement.

**Exemple 2: détection de la perte d'hétérozygotie sur trois régions chromosomiques indépendantes chez 52 patients**

La détection d'au moins une perte d'hétérozygotie a été recherchée sur trois et six régions chromosomiques prostate sur 52 patients âgés de 53 à 86 ans. Les résultats obtenus lors de l'analyse de trois et six régions chromosomiques sont présentés respectivement par l'exemple 2 et l'exemple 3.

**A : Matériels et méthodes**

**A1 : Prélèvement des cellules**

Les conditions de prélèvement des cellules sont identiques à celles décrites dans l'exemple 1 consacré à l'étude de 32 patients.

**A2 Extraction des ADN et ARN des cellules**

Les ADNs et ARNs des cellules d'origines prostatique ainsi que les ADNs des lymphocytes sanguins prélevés ont été extraits selon le même protocole que celui utilisé dans l'exemple 1.

Les réactions de PCR réalisées pour la quantification des ARN messagers du PSA et la détection des LOH sont identiques à celles citées dans l'exemple 1.

**B. Résultats**

Le tableau 4 ci-dessous présente l'ensemble des données obtenues dans cette étude sur chacun des 52 patients. Plus précisément, il présente le numéro d'identification anonyme de chaque patient (DKPRO), la détection d'au moins une perte d'hétérozygotie parmi les trois régions analysées (test à valeur diagnostic selon l'invention), les résultats de l'analyse de la biopsie réalisée après le diagnostic (cancer de la prostate ou non).

Tableau 4:

| présentation des résultats de LOH sur trois régions chromosomiques en analysant l'ADN de 52 patients | | | | | | | | | |
|---|---|---|---|---|---|---|---|---|---|
| Régions | 7q31 | | | 8p22 | | 13q14 | | | Analyse Biopsie |
| DKPRO | D7S480 | D7S522 | D7S523 | D8S261 | D8S1786 | D13S153 | D13S272 | D13S284 | |
| 3183 | LOH | LOH | LOH | + | + | + | + | + | non |
| 3202 | NI | LOH | + | NI | + | LOH | NI | ND | Cancer de la prostate |
| 3197 | + | + | + | + | + | + | + | + | non |
| 3182 | NI | NI | + | + | NI | + | + | + | non |
| 3198 | + | NI | + | + | + | + | NI | + | non |
| 3203 | + | + | + | LOH | + | + | + | + | Cancer de la prostate |
| 3194 | NI | NI | + | + | NI | + | + | + | Cancer de la prostate |
| 3190 | + | NI | LOH | NI | + | LOH | NI | + | Cancer de la prostate |
| 3193 | + | + | + | NI | + | + | NI | + | non |
| 3191 | + | NI | + | + | LOH | LOH | NI | + | non |
| 3195 | LOH | ND | + | LOH | NI | + | + | NI | Cancer de la prostate |
| 3196 | + | NI | + | LOH | + | + | + | + | Cancer de la prostate |
| 3199 | LOH | + | + | + | + | + | + | + | non |
| 3200 | + | NI | + | LOH | + | + | NI | + | non |
| 3220 | + | NI | + | + | NI | + | NI | + | Cancer de la prostate |
| 3215 | LOH | LOH | + | LOH | + | + | + | + | Cancer de la prostate |
| 3219 | NI | LOH | NI | NI | + | + | + | + | Cancer de la prostate |
| 3226 | + | NI | LOH | + | NI | + | + | NI | non |
| 3229 | + | NI | + | + | + | + | + | + | non |
| 3216 | + | NI | NI | LOH | NI | + | + | + | Cancer de la prostate |
| 3228 | + | NI | + | + | + | LOH | + | + | Cancer de la prostate |
| 3212 | + | NI | + | LOH | + | + | + | + | Cancer de la prostate |
| 3286 | + | + | LOH | + | NI | + | NI | + | non |

Tableau 4: (suite)

| présentation des résultats de LOH sur trois régions chromosomiques en analysant l'ADN de 52 patients | | | | | | | | | |
|---|---|---|---|---|---|---|---|---|---|
| Régions | 7q31 | | | 8p22 | | 13q14 | | | Analyse Biopsie |
| DKPRO | D7S480 | D7S522 | D7S523 | D8S261 | D8S1786 | D13S153 | D13S272 | D13S284 | |
| 3245 | + | + | + | + | + | + | + | + | non |
| 3285 | + | + | + | + | + | + | + | + | non |
| 3267 | + | + | + | + | + | + | NI | + | non |
| 3263 | NI | + | + | LOH | LOH | + | + | NI | non |
| 3283 | + | NI | + | + | LOH | NI | + | + | Cancer de la prostate |
| 3238 | + | NI | + | LOH | + | + | NI | NI | Cancer de la prostate |
| 3246 | + | + | + | + | + | + | NI | + | Cancer de la prostate |
| 3284 | + | + | + | + | + | + | + | + | non |
| 3282 | NI | + | + | + | LOH | - + | + | NI | non |
| 3278 | + | NI | + | + | + | + | + | + | non |
| 3277 | + | NI | NI | NI | + | + | + | ND | non |
| 3276 | + | LOH | + | + | NI | LOH | + | + | Cancer de la prostate |
| 3265 | + | + | + | + | + | NI | + | + | Cancer de la prostate |
| 3279 | + | NI | LOH | + | LOH | + | + | NI | Cancer de la prostate |
| 3249 | NI | NI | NI | + | + | + | NI | + | non |
| 3266 | + | NI | + | NI | LOH | + | NI | LOH | Cancer de la prostate |
| 3280 | NI | NI | LOH | + | + | + | + | + | non |
| 3250 | + | + | NI | + | + | NI | + | LOH | non |
| 3292 | ND | NI | + | + | + | NI | LOH | + | Cancer de la prostate |
| 3295 | LOH | LOH | + | NI | LOH | + | NI | + | Cancer de la prostate |
| 3293 | NI | + | + | + | LOH | + | + | + | Cancer de la prostate |
| 3241 | LOH | LOH | + | + | + | + | + | + | Cancer de la prostate |
| 3248 | LOH | NI | + | NI | + | + | + | LOH | Cancer de la prostate |

EP 1 294 941 B1

Tableau 4:   (suite)

| présentation des résultats de LOH sur trois régions chromosomiques en analysant l'ADN de 52 patients | | | | | | | | |
|---|---|---|---|---|---|---|---|---|
| Régions | 7q31 | | | 8p22 | | 13q14 | | | Analyse Biopsie |
| DKPRO | D7S480 | D7S522 | D7S523 | D8S261 | D8S1786 | D13S153 | D13S272 | D13S284 | |
| 3334 | ND | LOH | LOH | ND | + | LOH | + | + | Cancer de la prostate |
| 3325 | + | + | + | + | + | + | LOH | NI | Cancer de la prostate |
| 3330 | NI | NI | + | NI | + | + | NI | + | non |
| 3281 | + | LOH | + | + | + | + | + | + | Cancer de la prostate |
| 3329 | + | + | LOH | NI | + | + | NI | + | non |
| 3328 | + | + | + | + | + | + | NI | + | non |

L'annotation NI (non informatif) indique que le patient est homozygote pour la région analysée, ND (non détenniné) indique qu'aucun résultat de génotypage interprétable n'a été obtenu après au moins trois PCR successives, LOH et + indiquent respectivement la perte d'allèle et la présence de l'hétérozygotie

Le nombre de perte d'hétérozygotie détectée sur l'ensemble des trois régions chromosomiques analysées et pour chacun des deux groupes cliniques (cancer de la prostate ou non) de patients étudiés est détaillé dans le tableau 5 ci-dessous.

**Tableau 5 :    Présentation des données obtenues sur la détection de perte d'hétérozygotie sur chacune des trois régions indépendantes**

|  | Pas de cancer de la prostate | Cancer de la prostate | totaux |
|---|---|---|---|
| Détection de LOH | 11 patients | 23 patients | 34 patients |
| Pas de détection de LOH | 14 patients | 4 patients | 18 patients |
| totaux | 25 patients | 27 patients | |

Les paramètres de sensibilité, de spécificité et de valeurs prédictives positives et négatives du test LOH pour cette étude réalisée sur 52 patients sont présentés dans le tableau 6.

Tableau 6:

| Présentation des paramètres de sensibilité, de spécificité et de valeurs prédictives selon le test réalisé pour le diagnostic par le test LOH | | | |
|---|---|---|---|
| **Sensibilité** | **Spécificité** | **Valeur Prédictive** | |
| | | **négative** | **positive** |
| 85% | 56% | 77% | 67% |

Les résultats montrent que, bien que l'on observe une légère diminution de la valeur prédictive positive pour cette dernière étude, l'ensemble des paramètres est globalement conservé entre les séries d'analyses de l'exemple 1 et de l'exemple 2.

**Exemple 3 : détection de la perte d'hétérozygotie sur six régions chromosomiques indépendantes chez 52 patients**

La perte d'allèle a été recherchée chez les 52 patients présentés dans l'exemple 2 sur un nombre de régions chromosomiques porté à six et qui comprennent les trois régions de l'exemple 2 et les régions 12p13, 16q23.2 et 18q21.

**A : Matériels et méthodes**

**A1 : Prélèvement des cellules**

Les conditions de prélèvement des cellules sont identiques à celles décrites dans l'exemple 1 consacré à l'étude de 32 patients.

**A2 Extraction des ADN et ARN des cellules**

Les ADNs et ARNs des cellules d'origines prostatique ainsi que les ADNs des lymphocytes sanguins prélevés ont été extraits selon le même protocole que celui utilisé dans l'exemple 1. Les réactions de PCR réalisées pour la quantification des ARN messagers du PSA et la détection des LOH sont identiques à celles citées dans l'exemple 1.

**B. Résultats**

Le tableau 7 ci-dessous présente l'ensemble des données obtenues dans cette étude sur chacun des 52 patients. Plus précisément, il présente le numéro d'identification anonyme de chaque patient (DKPRO), la détection d'au moins une perte d'hétérozygotie parmi les six régions analysées (test à valeur diagnostic selon l'invention), les résultats de l'analyse de la biopsie réalisée après le diagnostic (cancer de la prostate ou non).

## <u>Tableau 7</u> : présentation des résultats de LOH sur six régions chromosomiques en analysant l'ADN de 52 patients

| Régions | 7q31 | | | 8p22 | | 12p13 | | 13q14 | | | 16q23.2 | | 18q21 | | Analyse Biopsie |
|---|---|---|---|---|---|---|---|---|---|---|---|---|---|---|---|
| DKPRO | D7 S480 | D7 S522 | D7 S523 | D8 S261 | D8 S1786 | D12 S77 | D12 S358 | D13 S153 | D13 S272 | D13 S284 | D16 S518 | D16 S507 | D18 S39 | D18 S46 | |
| 3183 | LOH | LOH | LOH | + | + | + | + | + | + | + | ND | ND | LOH | LOH | non |
| 3202 | NI | LOH | + | NI | + | LOH | LOH | LOH | NI | ND | + | NI | LOH | LOH | Cancer de la prostate |
| 3197 | + | + | + | + | + | + | + | + | + | + | + | + | NI | + | non |
| 3182 | NI | NI | + | + | NI | + | + | + | + | + | + | + | LOH | LOH | non |
| 3198 | + | NI | + | + | + | + | + | + | NI | + | + | + | NI | + | non |
| 3203 | + | + | + | LOH | + | + | + | + | + | + | LOH | LOH | + | + | Cancer de la prostate |
| 3194 | NI | NI | + | + | NI | ND | ND | + | + | + | LOH | ND | LOH | ND | Cancer de la prostate |
| 3190 | + | NI | LOH | NI | + | + | LOH | LOH | NI | + | + | + | + | + | Cancer de la prostate |
| 3193 | + | + | + | NI | + | + | + | + | NI | + | + | NI | + | NI | non |
| 3191 | + | NI | + | + | LOH | ND | ND | LOH | NI | + | + | ND | ND | ND | non |
| 3195 | LOH | ND | + | LOH | NI | ND | ND | + | + | NI | + | + | ND | ND | Cancer de la prostate |
| 3196 | + | NI | + | LOH | + | NI | LOH | + | + | + | + | NI | LOH | LOH | Cancer de la prostate |
| 3199 | LOH | + | + | + | + | + | + | + | + | + | + | NI | + | NI | non |
| 3200 | + | NI | + | LOH | + | NI | + | + | NI | + | LOH | LOH | LOH | LOH | non |
| 3220 | + | NI | + | + | NI | + | + | + | NI | + | + | + | + | + | Cancer de la prostate |
| 3215 | LOH | LOH | + | LOH | + | ND | LOH | + | + | + | LOH | LOH | LOH | NI | Cancer de la prostate |
| 3219 | NI | LOH | NI | NI | + | LOH | LOH | + | + | + | LOH | NI | NI | ND | Cancer de la prostate |
| 3226 | + | NI | LOH | + | NI | + | + | + | + | NI | LOH | + | NI | ND | non |
| 3229 | + | NI | + | + | + | + | + | + | + | + | + | + | + | + | non |
| 3216 | + | NI | NI | LOH | NI | + | LOH | + | + | + | + | NI | LOH | LOH | Cancer de la prostate |
| 3228 | + | NI | + | + | + | + | + | LOH | + | + | LOH | + | LOH | LOH | Cancer de la prostate |
| 3212 | + | NI | + | LOH | + | + | NI | + | + | + | + | + | + | NI | Cancer de la prostate |
| 3286 | + | + | LOH | + | NI | + | + | + | NI | + | + | ND | + | + | non |
| 3245 | + | + | + | + | + | + | + | + | + | + | + | + | + | + | non |
| 3285 | + | + | + | + | + | + | + | + | + | + | + | + | + | + | non |
| 3267 | + | + | + | + | + | + | + | + | NI | + | + | NI | + | + | non |
| 3263 | NI | + | + | LOH | LOH | + | NI | + | + | NI | + | ND | LOH | LOH | non |
| 3283 | + | NI | + | + | LOH | NI | + | NI | + | + | + | NI | + | + | Cancer de la prostate |
| 3238 | + | NI | + | LOH | + | LOH | LOH | + | NI | NI | LOH | NI | + | NI | Cancer de la prostate |
| 3246 | + | + | + | + | + | + | + | + | NI | + | + | + | + | + | Cancer de la prostate |
| 3284 | + | + | + | + | + | + | + | + | + | + | + | + | + | + | non |
| 3282 | NI | + | + | + | LOH | NI | + | + | + | NI | + | + | NI | NI | non |

| 3278 | + | NI | + | + | + | + | ND | + | + | + | + | + | + | + | non |
|---|---|---|---|---|---|---|---|---|---|---|---|---|---|---|---|
| 3277 | + | NI | NI | NI | + | + | NI | + | + | ND | LOH | NI | + | + | non |
| 3276 | + | LOH | + | + | NI | + | + | NI | + | + | + | ND | LOH | ND | Cancer de la prostate |
| 3265 | + | + | + | + | + | LOH | + | NI | + | + | + | + | LOH | + | Cancer de la prostate |
| 3279 | + | NI | LOH | + | LOH | + | + | + | + | NI | LOH | + | + | + | Cancer de la prostate |
| 3249 | NI | NI | NI | + | + | + | + | + | NI | + | + | + | NI | + | non |
| 3266 | + | NI | + | NI | LOH | LOH | LOH | + | NI | LOH | LOH | + | LOH | + | Cancer de la prostate |
| 3280 | NI | NI | LOH | + | + | NI | NI | + | + | + | LOH | + | NI | LOH | non |
| 3250 | + | + | NI | + | + | + | + | NI | + | LOH | LOH | LOH | LOH | NI | non |
| 3292 | ND | NI | + | + | + | + | LOH | NI | LOH | + | LOH | LOH | + | + | Cancer de la prostate |
| 3295 | LOH | LOH | + | NI | + | NI | LOH | + | NI | + | NI | NI | NI | + | Cancer de la prostate |
| 3293 | NI | + | + | + | LOH | LOH | LOH | + | + | + | + | + | LOH | + | Cancer de la prostate |
| 3241 | LOH | LOH | + | + | + | ND | NI | + | + | + | + | ND | LOH | ND | Cancer de la prostate |
| 3248 | LOH | NI | + | NI | + | LOH | ND | + | + | LOH | + | + | + | ND | Cancer de la prostate |
| 3334 | ND | LOH | LOH | ND | + | LOH | LOH | LOH | + | + | LOH | NI | ND | NI | Cancer de la prostate |
| 3325 | + | + | + | + | + | LOH | LOH | + | LOH | NI | LOH | NI | NI | NI | Cancer de la prostate |
| 3330 | NI | NI | + | NI | + | + | + | + | NI | + | + | + | LOH | + | non |
| 3281 | + | LOH | + | + | + | NI | + | + | + | + | + | + | + | ND | Cancer de la prostate |
| 3329 | + | + | LOH | NI | + | ND | LOH | + | NI | + | LOH | + | NI | ND | non |
| 3328 | + | + | + | + | + | + | NI | + | NI | + | + | + | + | non |

L'annotation NI (non informatif) indique que le patient est homozygote pour la région analysée, ND (non déterminé) indique qu'aucun résultat de génotypage interprétable n'a été obtenu après au moins trois PCR successives, LOH et + indiquent respectivement la perte d'allèle et la présence de l'hétérozygotie.

Le nombre de perte d'hétérozygotie détectée sur l'ensemble des six régions chromosomiques analysées et pour chacun des deux groupes cliniques (cancer de la prostate ou non) de patients étudiés est détaillé dans le tableau 8 ci-dessous.

## Tableau 8 : Présentation des données obtenues sur la détection de perte d'hétérozygotie sur chacune des six régions indépendantes

| | Pas de cancer de la prostate | Cancer de la prostate | totaux |
|---|---|---|---|
| Détection de LOH | 14 patients | 25 patients | 39 patients |
| Pas de détection de LOH | 11 patients | 2 patients | 13 patients |
| totaux | 25 patients | 27 patients | |

Les paramètres de sensibilité, de spécificité et de valeurs prédictives positives et négatives du test LOH pour cette étude réalisée sur 52 patients sont présentés dans le tableau 9.

Tableau 9:

| Présentation des paramètres de sensibilité, de spécificité et de valeurs prédictives selon le test réalisé pour le diagnostic par le test LOH | | | | |
|---|---|---|---|---|
| | Sensibilité | Spécificité | Valeur Prédictive | |
| | | | négative | positive |
| Exemple 2 | 85% | 56% | 77% | 67% |
| Exemple 3 | 92% | 44% | 84% | 64% |

Les résultats montrent que l'augmentation du nombre de régions chromosomiques étudiées portèe aux six régions régions 7q31, 8p22, 12p13, 13q14, 16q23.2 et 18q21 permet d'augmenter la sensibilité et la valeur prédictive négative du test LOH. La diminution de la spécificité assortie de la valeur prédictive positive peut s'expliquer par le fait que 20 à 50% des biopsies sont prélevées dans du tissu sain à coté de la tumeur.

Le procédé de l'invention et sa mise en oeuvre offre au clinicien et au patient un moyen de diagnostic, pronostic et suivi d'un traitement des cancers de la prostate qui présente de nombreux avantages parmi lesquels il faut citer :

- la fiabilité, améliorée par rapport aux dosages PSA, peut être augmentée avec le nombre de régions chromosomiques analysées ;
- la méthode non invasive - comparée aux biopsies - permet un suivi régulier tant en pronostic qu'en suivi thérapeutique, contribuant ainsi à l'augmentation de la fiabilité des résultats ,
- le procédé est mis en oeuvre sur des cellules similaires caractérisées par leurs marqueurs spécifiques ; ainsi, la comparaison dans le temps des résultats obtenus est significative de l'évolution de la pathologie et/ou de l'effet d'un traitement.
- la possibilité de réaliser une base de données accumulant l'ensemble des données relatives aux patients suivis et traités, et d'affiner ainsi les traitements à prodiguer à un patient par comparaison de son tableau clinique, des caractéristiques phénotypiques et génotypiques de ses cellules prostatiques avec les données constitutives de la base.

## BIBLIOGRAPHIE

1. Partin, P. R. et Oesterling, J. E. (1 994). The clinical usefulness of prostate specific antigen: update 1994. *J. Uroi*. **152:** 1358.
2. Christensson. A., Bjo6p21, 6q21, 7q31, 8p22, 1Oq23-25, 13q14, 16q22, 16q23, 16q24, 17q21 et 18q21rk, T., Nilsson, O., Dahlen, U., Matikainen, T., Cockett, A. T. K., Abrahamsson, P.A. et Lilja, H. (1993). Serum prostate specific antigen complexed to al-antichymotrypsin as an indicator of prostate cancer. *J. UroL* **150:** 100.
3. Clements, J.A. et al. (1999). Molecular détection of prostate cells in ejculate and urethral washings in men with suspected prostate cancer. *J. Uroi*. **161:** 1337-1343.
4. Latil, A., Cussenot, O., Fournier, G., Baron, J.-C. et Lidereau, R. (1995). Loss of heterozygosity at 7q31 is a frequent and early event in prostate cancer. *Clin. Cancer. Res.* 1: 1385-1389.
5. Latil, A., Cussenot, O., Fournier, G., Keltouma, D. et Lidereau, R. (1997). Loss of heterozygosity at chromosome 16q in prostate adenocarcinoma : Identification of three indépendant regions. *Cancer Res.* **57:** 1058-1062.
6. Latil, A., Fournier, G., Cussenot, 0. et Lidereau, R. (1996). Differential chromosome allelic imbalance in the progression of human prostate cancer. *J. Uroi*. **156:** 2079-2083.
7. Ueda, T. et al. (1999). Identification of a 1 cM region of common deletion on 13q14 associated with human prostate cancer. Genes, *chromosomes and cancer* **24:** 183-190.
8. Yin, Z., Spitz, M.R, Babaiab, R.J., Strom, S.S., Troncoso, P. et Kagan, J. (1999). Limiting the location of a putative human prostate cancer tumor suppressor gene at chromosome 13q14.3. *Oncogene* **18.-** 75767583.
9. Jenkins, R.B. et al. (1998). A molecular cytogenetic analysis of 7q31 in prostate cancer. *Cancer Res* **58:** 759-766.
10. Prasad, M.A., Trybus, T.M., Wojno, K.J. and Macoska, J.A. (1998). Homozygous and frequent deletion of proximal 8p séquences in human prostate cancers : Identification of a potentiel tumor suppressor gene site. *Genes, chromosome and cancer* **23:** 255-262.
11. MacGrogan, D., Levy, A., Bostwick, D., Wagner, M., Wells, D. et Bookstein R. (1994). Loss of chromosome arm 8p loci in prostate cancer: Mapping by quantitative allelic imbalance. Genes, chromosome and cancer 10: 151-159.
12. Trapman, J. et al. (1994). Loss of. heterozygosity of chromosome 8 microsatellite loci implicates a candidate tumor suppressor gene between the loci D8S87 and D8S133 in human prostate cancer. *Cancer Res* **54:** 6061-6064.
13. Latil, A. et al. (1999). Loss of heterozygosity at chromosome arm 13q and RBI status in human prostate cancer. Human pathology **30:** 809-815.
14. Chomczynski, Y. et Sacchi, N. (1 987). *Anal. Biochem.* **162:** 156-159.
15. Pollack JR, Perou CM, Alizadeh AA, Eisen MB, Pergamenschikov A, Williams CF, Jeffrey SS, Botstein D, Brown PO (1999). Genome-wide analysis of DNA copy-number changes using cDNA'microarrays. Nat *Genet* **23:** 41-6.
16. Shalon D, Smith SJ, Brown PO (1996). A DNA microarray system for analyzing complex DNA samples using two-color fluorescent probe hybridization. Genome Res 6: 639-45.

17. Dib, C., Faure, S., Fizames, C., Samson, D., Drouot, N., Vignal, A., Missasseau, P., Marc, S., Hazan, J., Seboun, E., Lathrop, M., Gyapay, G., Morissette, J., and Weissenbach, J. (1 996). ' A compréhensive genetic map of the human genome based on 5.264 microsatelites. *Nature,* **380:** 152-154.

18. Périnchery,G., Sasaki, M., Angan,A., Kumar,V., Carroll, P. and Dahiya,R. (2000). 'Deletion of Y-chromosome specific genes in human prostae cancer'. *J. Urol*., 163:1339-42.

19. Tomfohrde, J., Wood, S., Schertzer, M., Wagner, MJ., Wells, D.E., Parrish, J., Sadler, L.A., Blanton, S.H., Daiger, S.P., Wang, Z., Wilkie, P.J. and Weber, J.L. ( 1992). Human chromosome 8 linkage map based on short tandem repeat polymorphisme: Effect of genotyping errors. *Genomics*, *14:144*.

20. Sambrook, J., Fritsch, E.F., and Maniatis, T. (1 989). *Molecular Cloning: a Laboratory Manual*, 2 edn. Cold Spring Harbor, NY: Cold Spring Harbor Laboratory Press.

**Revendications**

1. Procédé de diagnostic *in vitro* et/ou de pronostic du cancer de la prostate, **caractérisé en ce qu'**à partir d'un échantillon constitué d'urine et de cellules d'origine prostatique :

   a) on détermine le taux de cellules d'origine prostatique contenues dans l'échantillon, soit par extraction de l'ARN total des cellules d'origine prostatique puis analyse des ARN de marqueurs spécifiques desdites cellules, soit par dosage direct d'antigènes spécifiques des cellules prostatiques dans l'urine ;
   b) après extraction des ADN totaux des cellules d'origine prostatique, on recherche les altérations chromosomiques détectées par pertes d'allèles.

2. Procédé selon la revendication 1, **caractérisé en ce que**, lorsque la détermination du taux de cellules d'origine prostatique est effectuée à partir de l'analyse des ARN de marqueurs spécifiques, l'ADN et l'ARN sont extraits séparément ou simultanément.

3. Procédé selon la revendication 1 dans lequel l'extraction des ADN et ARN des cellules d'origine prostatique est effectuée après centrifugation de l'échantillon et récupération du culot cellulaire ou autre tri cellulaire permettant d'isoler les cellules d'origine prostatique.

4. Procédé selon la revendication 1 dans lequel l'analyse des ARN de marqueurs spécifiques est réalisée par une méthode d'amplification quantitative des ARN correspondants.

5. Procédé selon la revendication 1 dans lequel les marqueurs spécifiques des cellules d'origine prostatique sont choisis parmi les marqueurs suivants: PSA, PSMA, PAP, KRT 18, la vimentine, KRT 5 et CD34.

6. Procédé selon la revendication 4 dans lequel les amorces utilisées pour l'amplification des ARN des marqueurs ont une séquence nucléotidique définie dans le tableau suivant :

| KRT 18 | 5'-TGA GAC GTA CAG TCC AGT CCT-3' |
| | 5'-GCT CCA TCT GTA GGG CGT AG-3' |
| PAP | 5'-CAT CTG GAA TCC TAT CCT ACT CTG-3' |
| | 5'-AGT TCT TGA AAA CGA GGG CA-3' |
| PSA | 5'-ACC AGA GGA GTT CTT GAC CCC AAA-3' |
| | 5'-CCC CAG AAT CAC CCG AGC AG-3' |
| CD34 | 5'-GTG TCT CAA CAT GGC AAT GAG G-3' |
| | 5'-ACA GAG GTC TGT GAC TGG ACA GAA-3' |
| KRT5 | 5'-CCG TGC CGC AGT TCT ATA TTC-3' |
| | 5'-GTG GGG ATT CTG TTT TGA TGA TT-3' |
| VIMENTINE | 5'-CCT TGA ACG CAA AGT GGA ATC-3' |
| | 5'-GAC ATG CTG TTC CTG AAT CTG AG-3' |
| RPLPO | 5'-GGC GAC CTG GAA GTC CAA CT-3' |
| | 5'-CCA TCA GCA CCA CAG CCT TC-3' |

**7.** Procédé selon la revendication 1, dans lequel au moins une protéine choisie dans le groupe comprenant PSA, PSMA, PAP, prostase est dosée directement dans l'urine contenue dans l'échantillon de cellules d'origine prostatique.

**8.** Procédé selon la revendication 1 dans lequel la recherche d'altération chromosomique est réalisée par amplification quantitative de matrices porteuses de marqueurs de polymorphismes.

**9.** Procédé selon la revendication 8 dans lequel des marqueurs de polymorphisme utilisés sont des marqueurs microsatellites.

**10.** Procédé selon l'une quelconque des revendications 1 à 9 dans lequel la perte d'allèle est recherchée sur au moins une région chromosomique choisie dans le groupe comprenant les régions 6p21-31, 6q21, 6q25.3, 7q31, 8p22, 9p21, 10q23-25, 12p13, 13q14, 16q24.3, 16q23.2, 17q21.2, 18q21 et Yq11-12.

**11.** Procédé selon la revendication 10, **caractérisé en ce que** la perte d'allèle est recherchée sur les 3 régions chromosomiques suivantes : 7q31, 8p22, 13q14.

**12.** Procédé selon la revendication 10, **caractérisé en ce que** la perte d'allèle est recherchée sur les 4 régions chromosomiques suivantes : 7q31, 8p22, 13q14, 16q23.2

**13.** Procédé selon la revendication 10, caratérisé en ce que la perte d'allèle est recherchée sur les 6 régions chromosomiques suivantes : 7q31, 8p22, 12p13, 13q14, 16q23.2, et 18q21.

**14.** Procédé selon la revendication 9 dans lequel les marqueurs utilisés sont :.

| REGION CHROMOSOMIQUE | MARQUEUR |
|---|---|
| 6p21-31 | D6S1645, D6S1611, D6S291 |
| 6q21 | D6S404, D6S302 |
| 6q25.3 | D6S3398, D6S440, D6S420 |

(suite)

| REGION CHROMOSOMIQUE | MARQUEUR |
|---|---|
| 7q31 | D7S523, D7S480, D7S522 |
| 8p22 | D8S1786, D8S133, D8S261 |
| 9p21 | D9S1846, D9S171, D9S169 |
| 10q23-25 | D10S1765, D10S2491, D10S541, D10S1237 D10S587, D10S1223 |
| 12p13 | D12S77, D12S358, D12S89 |
| 13q14 | D13S272, D13S153, D13S284 |
| 16q23.2 | D16S507, D16S518 |
| 16q24.3 | D16S3098, D16S413, D10S520 |
| 17q21.2 | D17S932, D17S800, D17S855 |
| 18q21 | D18S39, D18S46 |
| Yq11-12 | BPY1, SMCY, RBM1, BPY2 |

**15.** Procédé selon la revendication 1 dans lequel on fait suivre l'étape de recherche d'altération chromosomique, d'une étape de détection puis de quantification du marqueur de la neuropiline (NRP1).

**16.** Procédé selon la revendication 15 dans lequel les amorces utilisées pour l'amplification du marqueur de la neuropiline sont choisies parmi les séquences de nucléotides suivantes : 5'-AGT GTC TTG CAG AGC AGT GTC TCA-3' et 5'-TGG TGC TAT ACT GGG AAG AAG CTG T- 3'.

**17.** Kit pour le diagnostic ou/et le pronostic *in vitro* du cancer de la prostate selon le procédé objet de l'une des revendications 1 à 16 comprenant au moins un de chacun des éléments suivants :

   a) des amorces spécifiques permettant l'amplification des ARN de marqueurs spécifiques des cellules d'origine prostatique et/ou un réactif de dosage d'un antigène spécifique des cellules prostatiques;
   b) des amorces spécifiques permettant l'amplification des séquences porteuses des marqueurs de polymorphisme;
   c) les tampons et enzymes nécessaires aux réactions d'amplification, de marquage et d'hybridation ;

**18.** Kit selon la revendication 17, **caractérisé en ce qu'**il contient en outre des cibles pour l'hybridation spécifique des produits amplifiés et leur quantification ; lesdites cibles pouvant être fixées sur un support.

**19.** Kit selon la revendication 17 contenant des amorces permettant l'amplification spécifique des séquences porteuses des marqueurs de polymorphismes situées au niveau des régions 6p21-31, 6q21, 6q25.3, 7q31, 8p22, 9p21, 10q23-25, 12p13, 13q14, 16q24.3, 16q23.2, 17q21.2, 18q21 et Yq11-12,

**20.** Kit selon la revendication 17 contenant des amorces permettant l'amplification spécifique des séquences porteuses des marqueurs de polymorphismes situées au niveau des trois régions 7q31, 8p22 et 13q14

**21.** Kit selon la revendication 17 contenant des amorces permettant l'amplification spécifique des séquences porteuses des marqueurs de polymorphismes situées au niveau des quatre régions 7q31, 8p22, 13q14 et 16q23.2.

**22.** Kit selon la revendication 17 contenant des amorces permettant l'amplification spécifique des séquences porteuses des marqueurs de polymorphismes situées au niveau des six régions 7q31, 8p22, 12p13, 13q14, 16q23.2 et 18q21.

**23.** Kit selon l'une des revendications 17 à 22 contenant en outre un réactif de dosage dans l'urine d'au moins d'un antigène spécifique choisi parmi PSA, PSMA, PAP ou prostase.

**24.** Kit selon la revendication 17 à 23 contenant en outre des amorces pour l'amplification du marqueur de la neuropiline (NRP1), dont la séquence nucléotidique est choisie dans le tableau suivant *:* 5'-AGT GTC TTG CAG AGC AGT GTC TCA- 3' et 5'-TGG TGC TAT ACT GGG AAG AAG CTG T- 3'.

**25.** Kit selon la revendication 17 dans lequel les amorces permettant l'amplification spécifique de l'ARN des marqueurs spécifiques des cellules prostatiques ont une séquence nucléotidique définie dans le tableau suivant :

| KRT 18 | 5'-TGA GAC GTA CAG TCC AGT CCT-3' |
| | 5'-GCT CCA TCT GTA GGG CGT AG-3' |
| PAP | 5'-CAT CTG GAA TCC TAT CCT ACT CTG-3' |
| | 5'-AGT TCT TGA AAA CGA GGG CA-3' |
| PSA | 5'-ACC AGA GGA GTT CTT GAC CCC AAA-3' |
| | 5'-CCC CAG AAT CAC CCG AGC AG-3' |
| CD34 | 5'-GTG TCT CAA CAT GGC AAT GAG G-3' |
| | 5'-ACA GAG GTC TGT GAC TGG ACA GAA-3' |
| KRT5 | 5'-CCG TGC CGC AGT TCT ATA TTC-3' |
| | 5'-GTG GGG ATT CTG TTT TGA TGA TT-3' |
| VIMENTINE | 5'-CCT TGA ACG CAA AGT GGA ATC-3' |
| | 5'-GAC ATG CTG TTC CTG AAT CTG AG-3' |
| RPLPO | 5'-GGC GAC CTG GAA GTC CAA CT-3' |
| | 5'-CCA TCA GCA CCA CAG CCT TC-3' |

**Patentansprüche**

**1.** Verfahren zur *in vitro* Diagnose und/oder Prognose von Prostatakrebs, **dadurch gekennzeichnet, dass** ausgehend von einer Probe, welche aus Urin und Zellen prostatischen Ursprungs besteht,

a) der in der Probe enthaltene Gehalt an Zellen prostatischen Ursprungs bestimmt wird, entweder durch Extraktion der Gesamt-RNS der Zellen prostatischen Ursprungs und anschließender Analyse spezifischer RNS-Marker der Zellen oder durch direkte Mengenbestimmung spezifischer Antigene der Zellen prostatischen Ursprungs im Urin;
b) nach Extraktion der Gesamt-DNS der Zellen prostatischen Ursprungs auf chromosomale Veränderungen untersucht wird, die durch Allelverlust nachgewiesen werden.

**2.** Verfahren nach Anspruch 1, **dadurch gekennzeichnet, dass** bei Bestimmung des Gehalts an Zellen prostatischen Ursprungs durch Analyse spezifischer RNS-Marker, die DNS und die RNS getrennt voneinander oder gleichzeitig extrahiert werden.

**3.** Verfahren nach Anspruch 1, wobei die Extraktion von DNS und RNS der Zellen prostatischen Ursprungs nach Zentrifugation der Probe und Wiedergewinnung der Zellpellets oder nach einer anderen Zellsortierung, welche eine Isolierung der Zellen prostatischen Ursprungs ermöglicht, erfolgt.

**4.** Verfahren nach Anspruch 1, wobei die Analyse der spezifischen RNS-Marker mittels eines Verfahrens quantitativer Amplifikation der entsprechenden RNS erfolgt.

**5.** Verfahren nach Anspruch 1, wobei die spezifischen RNS-Marker der Zellen prostatischen Ursprungs aus den folgenden Markern ausgewählt werden: PSA, PSMA, PAP, KRT 18, Vimentin, KRT 5 und CD34.

6. Verfahren nach Anspruch 4, wobei die zur Amplifikation der RNS-Marker verwendeten Primer eine Nukleotidsequenz aufweisen, wie in der folgenden Tabelle angegeben:

| KRT 18 | 5'-TGA GAC GTA CAG TCC AGT CCT-3' |
| | 5'-GCT CCA TCT GTA GGG CGT AG-3' |
| PAP | 5'-CAT CTG GAA TCC TAT CCT ACT CTG-3' |
| | 5'-AGT TCT TGA AAA CGA GGG CA-3' |
| PSA | 5'-ACC AGA GGA GTT CTT GAC CCC AAA-3' |
| | 5'-CCC CAG AAT CAC CCG AGC AG-3' |
| CD34 | 5'-GTG TCT CAA CAT GGC AAT GAG G-3' |
| | 5'-ACA GAG GTC TGT GAC TGG ACA GAA-3' |
| KRT 5 | 5'-CCG TGC CGC AGT TCT ATA TTC-3' |
| | 5'-GTG GGG ATT CTG TTT TGA TGA TT-3' |
| VIMENTIN | 5'-CCT TGA ACG CAA AGT GGA ATC-3' |
| | 5'-GAC ATG CTG TTC CTG AAT CTG AG-3' |
| RPLPO | 5'-GGC GAC CTG GAA GTC CAA CT-3' |
| | 5'-CCA TCA GCA CCA CAG CCT TC-3' |

7. Verfahren nach Anspruch 1, wobei wenigstens ein Protein ausgewählt aus der PSA, PSMA, PAP, und Prostase umfassenden Gruppe direkt im Urin, der in der Probe von Zellen prostatischen Ursprungs enthalten ist, bestimmt wird.

8. Verfahren nach Anspruch 1, wobei die Untersuchung auf chromosomale Veränderungen durch quantitative Amplifikation von Trägermatrices von Polymorphismenmarkern erfolgt.

9. Verfahren nach Anspruch 8, wobei die verwendeten Polymorphismenmarker Mikrosatellitenmarker sind.

10. Verfahren nach einem der Ansprüche 1 bis 9, wobei der Allelverlust auf wenigstens einem der chromosomalen Bereiche ausgewählt aus der Gruppe, welche die Bereiche 6p21-31, 6q21, 6q25.3, 7q31, 8p22, 9p21, 10q23-25, 12p13, 13q14, 16q24.3, 16q23.2, 17q21.2, 18q21 und Yq11-12 umfasst, untersucht wird.

11. Verfahren nach Anspruch 10, **dadurch gekennzeichnet, dass** der Allelverlust auf den folgenden 3 chromosomalen Bereichen untersucht wird: 7q31, 8p22, 13q14.

12. Verfahren nach Anspruch 10, **dadurch gekennzeichnet, dass** der Allelverlust auf den folgenden 4 chromosomalen Bereichen untersucht wird: 7q31, 8p22, 13q14, 16q23.2.

13. Verfahren nach Anspruch 10, **dadurch gekennzeichnet, dass** der Allelverlust auf den folgenden 6 chromosomalen Bereichen untersucht wird: 7q31, 8p22, 12p13, 13q14, 16q23.2 und 18q21.

14. Verfahren nach Anspruch 9, wobei die verwendeten Marker sind:

| CHROMOSOMALER BEREICH | MARKER |
|---|---|
| 6p21-31 | D6S1645, D6S1611, D6S291 |
| 6q21 | D6S404, D6S302 |
| 6q25.3 | D6S3398, D6S440, D6S420 |
| 7q31 | D7S523, D7S480, D7S522 |
| 8p22 | D8S1786, D8S133, D8S261 |
| 9p21 | D9S1846, D9S171, D9S169 |
| 10q23-25 | D10S1765, D10S2491, D10S541, D10S1237, D10S587, D10S1223 |
| 12p13 | D12S77, D12S358, D12S89 |
| 13q14 | D13S272, D13S153, D13S284 |
| 16q23.2 | D16S507, D16S518 |
| 16q24.3 | D16S3098, D16S413, D10S520 |
| 17q21.2 | D17S932, D17S800, D17S855 |
| 18q21 | D18S39, D18S46 |
| Yq11-12 | BPY1, SMCY, RBMI, BPY2 |

**15.** Verfahren nach Anspruch 1, wobei dem Schritt der Untersuchung auf chromosomale Veränderung ein Schritt des Nachweises und anschließender Quantifizierung des Neuropilin-Markers (NRP 1) folgt.

**16.** Verfahren nach Anspruch 15, wobei die zur Amplifikation des Neuropilin-Markers verwendeten Primer aus den folgenden Nukleotidsequenzen ausgewählt werden: 5'-AGT GTC TTG CAG AGC AGT GTC TCA-3' und 5'-TGG TGC TAT ACT GGG AAG AAG CTG T-3'.

**17.** Kit zur *in vitro* Diagnose und/oder Prognose von Prostatakrebs gemäß dem Verfahren nach einem der Ansprüche 1 bis 16, der wenigstens je einen der folgenden Bestandteile umfasst

a) spezifische Primer, welche die Amplifikation spezifischer RNS-Marker von Zellen prostatischen Ursprungs ermöglichen und/oder ein Reagens zur Bestimmung der Menge eines spezifischen Antigens prostatischer Zellen;
b) spezifische Primer, welche die Amplifikation von Trägersequenzen von Polymorphismenmarkern ermöglichen;
c) die für die Reaktionen der Amplifikation, Markierung und Hybridisierung erforderlichen Puffer und Enzyme.

**18.** Kit nach Anspruch 17, **dadurch gekennzeichnet, dass** er des Weiteren Zielmoleküle zur spezifischen Hybridisierung der amplifizierten Produkte und deren Quantifizierung enthält, wobei die Zielmoleküle auf einem Träger befestigt sein können.

**19.** Kit nach Anspruch 17, der Primer enthält, welche die spezifische Amplifikation von Trägersequenzen von Polymorphismenmarkern ermöglichen, die auf den Bereichen 6p21-31, 6q21, 6q25.3, 7q31, 8p22, 9p21, 10q23-25, 12p13, 13q14, 16q24.3, 16q23.2, 17q21.2, 18q21 und Yq11-12 liegen.

**20.** Kit nach Anspruch 17, der Primer enthält, welche die spezifische Amplifikation von Trägersequenzen von Polymorphismenmarkern ermöglichen, die auf den drei Bereichen 7q31, 8p22 und 13q14 liegen.

**21.** Kit nach Anspruch 17, der Primer enthält, welche die spezifische Amplifikation von Trägersequenzen von Polymorphismenmarkern ermöglichen, die auf den vier Bereichen 7q31, 8p22, 13q14 und 16q23.2 liegen.

**22.** Kit nach Anspruch 17, der Primer enthält, welche die spezifische Amplifikation von Trägersequenzen von Polymorphismenmarkern ermöglichen, die auf den sechs Bereichen 7q31, 8p22, 12p13, 13q14, 16q23.2 und 18q21 liegen.

**23.** Kit nach einem der Ansprüche 17 bis 22, der ferner ein Reagens zur Bestimmung der Menge wenigstens eines spezifischen Antigens ausgewählt aus PSA, PSMA, PAP oder Prostase im Urin enthält.

**24.** Kit nach Anspruch 17 bis 23, der ferner Primer zur Amplifikation des Neuropilin-Markers (NRP 1) enthält, deren Nukleotidsequenz aus der folgenden Tabelle ausgewählt ist: 5'-AGT GTC TTG CAG AGC AGT GTC TCA-3' und 5'-TGG TGC TAT ACT GGG AAG AAG CTG T-3'.

**25.** Kit nach Anspruch 17, wobei die Primer, welche die spezifische Amplifikation der spezifischen RNS-Marker von prostatischen Zellen ermöglichen, eine Nukleotidsequenz aufweisen, wie in der folgenden Tabelle angegeben

| KRT 18 | 5'-TGA GAC GTA CAG TCC AGT CCT-3' |
| | 5'-GCT CCA TCT GTA GGG CGT AG-3' |
| PAP | 5'-CAT CTG GAA TCC TAT CCT ACT CTG-3' |
| | 5'-AGT TCT TGA AAA CGA GGG CA-3' |
| PSA | 5'-ACC AGA GGA GTT CTT GAC CCC AAA-3' |
| | 5'-CCC CAG AAT CAC CCG AGC AG-3' |
| CD34 | 5'-GTG TCT CAA CAT GGC AAT GAG G-3' |
| | 5'-ACA GAG GTC TGT GAC TGG ACA GAA-3' |
| KRT 5 | 5'-CCG TGC CGC AGT TCT ATA TTC-3' |
| | 5'-GTG GGG ATT CTG TTT TGA TGA TT-3' |
| Vimentin | 5'-CCT TGA ACG CAA AGT GGA ATC-3' |
| | 5'-GAC ATG CTG TTC CTG AAT CTG AG-3' |
| RPLPO | 5'-GGC GAC CTG GAA GTC CAA CT-3' |
| | 5'-CCA TCA GCA CCA CAG CCT TC-3' |

**Claims**

**1.** Method for *in vitro* diagnosis and/or prognosis of prostatic cancer, **characterized in that**, from a sample consisting of urine and of cells of prostatic origin:

a) the amount of cells of prostatic origin contained in the sample is determined either by extraction of the total RNA of the cells of prostatic origin and then analysis of the RNAs of markers specific for said cells, or by direct assaying of antigens specific for the prostatic cells in the urine;
b) after extraction of the total DNA of the cells of prostatic origin, chromosomal alterations detected by allelic losses are sought.

**2.** Method according to Claim 1, **characterized in that**, when the determination of the amount of cells of prostatic origin is carried out by means of the analysis of the RNAs of specific markers, the DNA and the RNA are extracted separately or simultaneously.

**3.** Method according to Claim 1, in which the extraction of the DNAs and RNAs of the cells of prostatic origin is carried out after centrifugation of the sample and recovery of the cell pellet, or other cell sorting making it possible to isolate the cells of prostatic origin.

4. Method according to Claim 1, in which the analysis of the RNAs of specific markers is carried out by a method of quantitative amplification of the corresponding RNAs.

5. Method according to Claim 1, in which the markers specific for the cells of prostatic origin are chosen from the following markers: PSA, PSMA, PAP, KRT 18, vimentin, KRT 5 and CD34.

6. Method according to Claim 4, in which the primers used for amplifying the RNAs of the markers have a nucleotide sequence defined in the table below:

| KRT 18 | 5'-TGA GAC GTA CAG TCC AGT CCT-3' |
| | 5'-GCT CCA TCT GTA GGG CGT AG-3' |
| PAP | 5'-CAT CTG GAA TCC TAT CCT ACT CTG-3' |
| | 5'-AGT TCT TGA AAA CGA GGG CA-3' |
| PSA | 5'-ACC AGA GGA GTT CTT GAC CCC AAA-3' |
| | 5'-CCC CAG AAT CAC CCG AGC AG-3' |
| CD34 | 5'-GTG TCT CAA CAT GGC AAT GAG G-3' |
| | 5'-ACA GAG GTC TGT GAC TGG ACA GAA-3' |
| KRT5 | 5'-CCG TGC CGC AGT TCT ATA TTC-3' |
| | 5'-GTG GGG ATT CTG TTT TGA TGA TT-3' |
| VIMENTIN | 5'-CCT TGA ACG CAA AGT GGA ATC-3' |
| | 5'-GAC ATG CTG TTC CTG AAT CTG AG-3' |
| RPLPO | 5'-GGC GAC CTG GAA GTC CAA CT-3' |
| | 5'-CCA TCA GCA CCA CAG CCT TC-3' |

7. Method according to Claim 1, in which at least one protein chosen from the group comprising PSA, PSMA, PAP and prostase is assayed directly in the urine contained in the sample of cells of prostatic origin.

8. Method according to Claim 1, in which the search for chromosomal alteration is carried out by quantitative amplification of matrices carrying polymorphism markers.

9. Method according to Claim 8, in which polymorphism markers used are microsatellite markers.

10. Method according to any one of Claims 1 to 9, in which the allelic loss is sought on at least one chromosomal region chosen from the group comprising the regions 6p21-31, 6q21, 6q25.3, 7q31, 8p22, 9p21, 10q23-25, 12p13, 13q14, 16q24.3, 16q23.2, 17q21.2, 18q21 and Yq11-12.

11. Method according to Claim 10, **characterized in that** the allelic loss is sought on the following 3 chromosomal regions: 7q31, 8p22 and 13q14.

12. Method according to Claim 10, **characterized in that** the allelic loss is sought on the following 4 chromosomal regions: 7q31, 8p22, 13q14 and 16q23.2.

13. Method according to Claim 10, **characterized in that** the allelic loss is sought on the following 6 chromosomal regions: 7q31, 8p22, 12p13, 13q14, 16q23.2 and 18q21.

14. Method according to Claim 9, in which the markers used are:

| CHROMOSOMAL REGION | MARKER |
| --- | --- |
| 6p21-31 | D6S1645, D6S1611, D6S291 |

(continued)

| CHROMOSOMAL REGION | MARKER |
|---|---|
| 6q21 | D6S404, D6S302 |
| 6q25.3 | D6S3398, D6S440, D6S420 |
| 7q31 | D7S523, D7S480, D7S522 |
| 8p22 | D8S1786, D8S133, D8S261 |
| 9p21 | D9S1846, D9S171, D9S169 |
| 10q23-25 | D10S1765, D10S2491, D10S541, D10S1237 D10S587, D10S1223 |
| 12p13 | D12S77, D12S358, D12S89 |
| 13q14 | D13S272, D13S153, D13S284 |
| 16q23.2 | D16S507, D16S518 |
| 16q24.3 | D16S3098, D16S413, D10S520 |
| 17q21.2 | D17S932, D17S800, D17S855 |
| 18q21 | D18S39, D18S46 |
| Yq11-12 | BPY1, SMCY, RBMI, BPY2 |

15. Method according to Claim 1, in which the step consisting in searching for a chromosomal alteration is followed by a step consisting of detection and then of quantification of the neuropilin (NRP1) marker.

16. Method according to Claim 15, in which the primers used for amplifying the neuropilin marker are chosen from the following nucleotide sequences:

5'-AGT GTC TTG CAG AGC AGT GTC TCA- 3'

and

5'-TGG TGC TAT ACT GGG AAG AAG CTG T-3'.

17. Kit for the in vitro diagnosis and/or prognosis of prostatic cancer according to the method which is the subject of one of Claims 1 to 16, comprising at least one of each of the following elements:

a) specific primers for amplifying the RNAs of markers specific for the cells of prostatic origin and/or a reagent for assaying an antigen specific for the prostatic cells;
b) specific primers for amplifying the sequences carrying the polymorphism markers;
c) the buffers and enzymes required for the amplification, labelling and hybridization reactions.

18. Kit according to Claim 17, **characterized in that** it also contains targets for the specific hybridization of the amplified products and their quantification, it being possible for said targets to be attached to a support.

19. Kit according to Claim 17, containing primers for the specific amplification of the sequences carrying the polymorphism markers located in the regions 6p21-31, 6q21, 6q25.3, 7q31, 8p22, 9p21, 10q23-25, 12p13, 13q14, 16q24.3, 16q23.2, 17q21.2, 18q21 and Yq11-12.

20. Kit according to Claim 17, containing primers for the specific amplification of the sequences carrying the polymorphism markers located in the three regions 7q31, 8p22 and 13q14.

21. Kit according to Claim 17, containing primers for the specific amplification of the sequences carrying the polymorphism markers located in the four regions 7q31, 8p22, 13q14 and 16q23.2.

**22.** Kit according to Claim 17, containing primers for the specific amplification of the sequences carrying the polymorphism markers located in the six regions 7q31, 8p22, 12p13, 13q14, 16q23.2 and 18q21.

**23.** Kit according to one of Claims 17 to 22, also containing a reagent for assaying in the urine at least one specific antigen chosen from PSA, PSMA, PAP and prostase.

**24.** Kit according to Claims 17 to 23, also containing primers for amplifying the neuropilin (NRP1) marker, the nucleotide sequence of which is chosen from the following table:

<div align="center">

5'-AGT  GTC  TTG  CAG  AGC  AGT  GTC  TCA-  3'

</div>

and

<div align="center">

5'-TGG TGC TAT ACT GGG AAG AAG CTG T-3'.

</div>

**25.** Kit according to Claim 17, in which the primers for the specific amplification of the RNAs of the markers specific for the prostatic cells have a nucleotide sequence defined in the following table:

| KRT 18 | 5'-TGA GAC GTA CAG TCC AGT CCT-3' |
| | 5'-GCT CCA TCT GTA GGG CGT AG-3' |
| PAP | 5'-CAT CTG GAA TCC TAT CCT ACT CTG-3' |
| | 5'-AGT TCT TGA AAA CGA GGG CA-3' |
| PSA | 5'-ACC AGA GGA GTT CTT GAC CCC AAA-3' |
| | 5'-CCC CAG AAT CAC CCG AGC AG-3' |
| CD34 | 5'-GTG TCT CAA CAT GGC AAT GAG G-3' |
| | 5'-ACA GAG GTC TGT GAC TGG ACA GAA-3' |
| KRT5 | 5'-CCG TGC CGC AGT TCT ATA TTC-3' |
| | 5'-GTG GGG ATT CTG TTT TGA TGA TT-3' |
| VIMENTIN | 5'-CCT TGA ACG CAA AGT GGA ATC-3' |
| | 5'-GAC ATG CTG TTC CTG AAT CTG AG-3' |
| RPLPO | 5'-GGC GAC CTG GAA GTC CAA CT-3' |
| | 5'-CCA TCA GCA CCA CAG CCT TC-3' |